(19) Europäisches Patentamt / European Patent Office / Office européen des brevets

(11) **EP 2 069 777 B1**

(12) **EUROPÄISCHE PATENTSCHRIFT**

(45) Veröffentlichungstag und Bekanntmachung des Hinweises auf die Patenterteilung:
**01.12.2010 Patentblatt 2010/48**

(51) Int Cl.:
*G01N 33/36* (2006.01)    *G01N 22/00* (2006.01)

(21) Anmeldenummer: **07820777.6**

(22) Anmeldetag: **01.10.2007**

(86) Internationale Anmeldenummer:
**PCT/EP2007/060393**

(87) Internationale Veröffentlichungsnummer:
**WO 2008/040704 (10.04.2008 Gazette 2008/15)**

(54) **VORRICHTUNG FÜR EINE TEXTILMASCHINE ZUR MESSUNG DER LÄNGENSPEZIFISCHEN MASSE UND/ODER DER FEUCHTIGKEIT EINES STRANGFÖRMIGEN, LAUFENDEN FASERGEMENGES**

DEVICE FOR A TEXTILE MACHINE FOR MEASURING THE LENGTH-SPECIFIC MASS AND/OR THE MOISTURE OF A STRAND-SHAPED, CONTINUOUS FIBER BATCH

DISPOSITIF POUR UNE MACHINE TEXTILE DESTINÉ À MESURER LE POIDS SPÉCIFIQUE À LA LONGUEUR ET/OU L'HUMIDITÉ D'UNE QUANTITÉ DE FIBRE DÉFILANTE EN ÉCHEVEAU

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **02.10.2006 DE 102006046714**

(43) Veröffentlichungstag der Anmeldung:
**17.06.2009 Patentblatt 2009/25**

(73) Patentinhaber: **Rieter Ingolstadt GmbH**
**85055 Ingolstadt (DE)**

(72) Erfinder: **RUSSER, Peter**
**81929 München (DE)**

(74) Vertreter: **Schlief, Thomas P.**
**Patentanwälte**
**Canzler & Bergmeier**
**Friedrich-Ebert-Straße 84**
**85055 Ingolstadt (DE)**

(56) Entgegenhaltungen:
EP-A- 0 288 135     WO-A-03/050530
DE-A1- 19 705 260     US-B1- 6 476 619

**EP 2 069 777 B1**

## Beschreibung

Die vorliegende Erfindung betrifft eine Vorrichtung für eine Textilmaschine zur Messung der längenspezifischen Masse und/oder der Feuchtigkeit eines strangförmigen, laufenden Fasergemenges. Eine erfindungsgemäße Vorrichtung ist insbesondere für eine Spinnereivorbereitungsmaschine, beispielsweise für eine Strecke, Karde oder Kämmmaschine oder für eine Spinnereimaschine, beispielsweise für eine Spinnmaschine oder Spulmaschine vorgesehen. Sie umfasst einen Mikrowellenresonator, welcher einen Messraum aufweist, durch den das Fasermenge hindurchführbar ist, und eine Anordnung zur Erfassung der Resonanzfrequenz und/oder der Güte des Mikrowellenresonators.

Weiterhin betrifft die vorliegende Erfindung eine Textilmaschine, insbesondere eine Spinnereivorbereitungsmaschine, beispielsweise eine Strecke, Karde oder Kämmmaschine oder eine Spinnereimaschine, beispielsweise eine Spinnmaschine oder Spulmaschine, mit besagter Vorrichtung.

Im Betrieb einer Textilmaschine ist es häufig erforderlich, ein strangförmiges, laufendes Fasergemenge hinsichtlich seiner längenspezifischen Masse zu vermessen. Der Begriff "strangförmiges Fasergemenge" umfasst dabei beispielsweise Faserbänder, Garne oder Zwirne. Die längenspezifische Masse gibt dabei an, welche Masse das Fasergemenge pro Längeneinheit aufweist. Die Messergebnisse dieser Größe werden insbesondere zur Steuerung der jeweiligen Textilmaschine herangezogen. Weiterhin dienen derartige Messergebnisse zur Beurteilung der Qualität eines produzierten Fasergemenges. Die bisher überwiegend eingesetzten, mechanisch abtastenden Sensoren zur Ermittlung der längenspezifischen Masse von laufenden Fasergemengen werden den Anforderungen an künftige Generationen von Textilmaschinen, insbesondere wegen der angestrebten Erhöhung der Arbeitsgeschwindigkeit, nicht mehr gerecht.

Eine neue Methode zur Messung der längenspezifischen Masse eines Fasergemenges stellt hingegen die Verwendung von Mikrowellen dar. Dabei wird das zu vermessende Fasergemenge durch einen Messraum eines Mikrowellenresonators kontinuierlich hindurchgeführt. Da die dielektrischen Eigenschaften textiler Fasergemenge von den dielektrischen Eigenschaften der Umgebungsluft abweichen, verändert sich durch das Hindurchführen des Fasergemenges sowohl die Resonanzfrequenz als auch die Güte des Mikrowellenresonators.

Zur Bestimmung der Resonanzfrequenz und der Güte eines Mikrowellenresonators ist es bekannt (z.B. aus US6476619) ein mittels eines Mikrowellengenerators erzeugtes Mikrowellensignal mit einer variierenden Frequenz in den Mikrowellenresonator einzukoppeln. Üblicherweise wird dabei ein vorab festgelegter Frequenzbereich kontinuierlich oder in festgelegten Schritten durchfahren, wobei für jede Frequenz des eingekoppelten Mikrowellensignals die Amplitude der resultierenden Schwingung im Mikrowellenresonator erfasst wird.

Aus der so aufgenommenen Resonanzkurve kann dann sowohl die Resonanzfrequenz (das ist diejenige Frequenz, bei der die höchste Amplitude auftritt) als auch die Güte des Mikrowellenresonators ermittelt werden. Hierzu wird zunächst die Bandbreite des Mikrowellenresonators aus der Resonanzkurve bestimmt. Dabei gilt folgende Festsetzung:

die Bandbreite ist jener Frequenzbereich der Resonanzkurve, in dem die Amplitude größer oder gleich dem $1/\sqrt{2}$ -ten Teil der maximalen Amplitude ist. Die Güte kann dann in einfacher Weise bestimmt werden, indem die Resonanzfrequenz durch die Bandbreite geteilt wird.

Bei der Produktion und/oder der Verarbeitung von textilen Fasergemengen ist vor allem die Trocken- bzw. die Substanzmasse des Fasergemenges von Bedeutung. Allerdings neigen textile Fasergemenge, insbesondere wenn sie Naturfasern, wie beispielsweise Baumwollfasern, enthalten, zur Aufnahme von Feuchtigkeit. Sowohl die Trockenmasse als auch die Feuchtigkeit beeinflussen die Resonanzfrequenz und die Güte des Mikrowellenresonators. Da dies jedoch in unterschiedlicher Weise geschieht, kann aus der gemessenen Resonanzfrequenz und aus der gemessenen Güte des Mikrowellenresonators sowohl die Trockenmasse des Fasergemenges als auch dessen Feuchtegehalt ermittelt werden. Zur Klarstellung sei angemerkt, dass der hier verwendete Begriff "längenspezifische Masse" sich auf die absolute längenspezifische Trockenmasse bezieht, während sich der Begriff "Feuchtigkeit" auf die absolute längenspezifische Masse des vom Fasergemenge aufgenommenen Wassers bezieht.

Bei dem bekannten Verfahren entspricht die Messzeit mindestens jener Zeit, welche für das Durchfahren des vorab festgelegten Frequenzbereichs benötigt wird. Dabei kann der Frequenzbereich allerdings nicht beliebig schnell durchfahren werden, da die Amplitude des Mikrowellenresonators bei einer Änderung der Frequenz des Oszillators erst nach Abklingen des sogenannten Einschwingvorgangs ihren endgültigen Wert annimmt. Die minimal erforderliche Messzeit kann beispielsweise bei einem linearen Frequenzhub abgeschätzt werden, indem der zu durchfahrende Frequenzbereich durch das Quadrat der Bandbreite des Mikrowellenresonators geteilt wird. Die erforderliche Messzeit erhöht sich jedoch noch weiter, wenn die Genauigkeitsanforderungen steigen.

Wenn jedoch die Messzeit so erhöht wird, dass Einschwingvorgänge keine wesentliche Rolle mehr spielen, so beziehen sich die Amplitudenwerte der ermittelten Resonanzkurve bei einem laufenden Fasergemenge auf unterschiedliche Abschnitte desselben. Dies wiederum führt zu Ungenauigkeiten, welche mit steigender Laufgeschwindigkeit des Fasergemenges zunehmen.

Nachteilig bei dem bekannten Verfahren ist es somit, dass, insbesondere bei einem schnell laufenden Fasergemenge, die gewünschte Genauigkeit der Erfassung der längenspezifischen Masse eines Fasergemenges und/oder dessen aufgenommener Feuchtigkeit prinzipiell nicht erreicht werden kann.

Aufgabe der vorliegenden Erfindung ist es somit, eine Vorrichtung für eine Textilmaschine bzw. eine Textilmaschine vorzuschlagen, bei welcher die genannten Nachteile vermieden sind. Insbesondere soll der apparative Aufwand verringert und gleichzeitig die Genauigkeit der Erfassung der längenspezifischen Masse eines Fasergemenges und/oder dessen aufgenommener Feuchtigkeit verbessert werden.

Die Aufgabe wird gelöst durch die Merkmale des unabhängigen Anspruchs.

Bei einer erfindungsgemäßen Vorrichtung ist ein Signalgenerator vorgesehen, der zur Erzeugung eines Generatorsignals ausgebildet ist, dessen Spektrum mehrere Frequenzen umfasst. Das Generatorsignal ist in den Mikrowellenresonator eingekoppelt und ein aus dem Mikrowellenresonator ausgekoppeltes Ausgangssignal ist einer Auswerteschaltung zugeführt. Dabei ist die Auswerteschaltung zur simultanen Bestimmung der Amplitude und/oder der Phase mehrerer der besagten Frequenzen im Spektrum des Ausgangssignals ausgebildet.

Ein für Messungen an Tabak vorgesehener Mikrowellen-resonator, der mit einem aus mehreren Frequenzen bestehenden Signal gespeist wird, ist aus DE 19705260 bekannt.

Aus den so gleichzeitig erfassten Amplituden- und/oder Phasenübertragungswerten können dann die Resonanzfrequenz und/oder die Güte des Mikrowellenresonators rechnerisch bestimmt werden. Hieraus können dann in bekannter Weise Messwerte der längenspezifischen Masse und/oder der Feuchtigkeit des strangförmigen durch den Messraum hindurch geführten Fasergemenges erzeugt werden.

Anders als aus dem Stand der Technik bekannt, ist es jedoch nicht erforderlich, eine Resonanzkurve zu erfassen, indem nacheinander für verschiedene Frequenzen Amplituden- und/oder Phasenübertragungswerte gemessen und ausgewertet werden. Hierdurch verkürzt sich der Zeitbedarf zur Erzeugung eines Messwertes beträchtlich, so dass insbesondere mit steigender Laufgeschwindigkeit des Fasergemenges die Genauigkeit des jeweiligen Messwertes erhöht werden kann. Der einzelne Messwert wird nicht mehr durch die Bewegung des Abschnittes des Fasergemenges während der Messdauer verfälscht.

Vorteilhafterweise ist ein Synchronsignal vorgesehen, um die Auswerteschaltung mit dem Signalgenerator zu synchronisieren. Das Synchronsignal korrespondiert mit dem Generatorsignal, so dass das Übertragungsverhalten des Mikrowellenresonators exakt durch die Auswerteschaltung ermittelbar ist.

Bevorzugt ist der Signalgenerator so ausgebildet, dass das Spektrum des Generatorsignals durch eine vorgebbare kleinste Frequenz und/oder durch eine vorgebbare größte Frequenz begrenzt ist. Die Erzeugung eines derartigen einseitig oder zweiseitig bandbegrenzten Spektrums bietet den Vorteil, dass der Energiegehalt des Generatorsignals auf einen zur Auswertung vorgesehenen Frequenzbereich konzentriert werden kann.

Bevorzugt ist hierbei vorgesehen, dass die kleinste Frequenz kleiner als die minimale betriebliche Resonanzfrequenz des Mikrowellenresonators und/oder dass die größte Frequenz größer als die maximale Resonanzfrequenz des Mikrowellenresonators ist. Die maximale Resonanzfrequenz des Mikrowellenresonators ist jene Resonanzfrequenz, welche sich bei leerem Messraum einstellt. Die minimale betriebliche Resonanzfrequenz hingegen ist jene Resonanzfrequenz, welche sich bei maximal gefülltem Messraum ergibt. Der Frequenzbereich zwischen minimaler Resonanzfrequenz und maximaler Resonanzfrequenz wird auch Abstimmbereich bezeichnet. Wenn nun die Breite des Spektrums den Abstimmbereich überdeckt, so ist es bei jedem Füllgrad des Messraumes des Mikrowellenresonators möglich, Frequenzen in der Nähe der Resonanzfrequenz auszuwerten. Dies verbessert letztendlich die Genauigkeit der Messungen.

Es ist zwar denkbar, dass der Signalgenerator zur Erzeugung eines Generatorsignals mit einem kontinuierlichen Spektrum ausgebildet ist, bevorzugt ist es allerdings, wenn der Signalgenerator so ausgebildet ist, dass die besagten Frequenzen im Spektrum des Generatorsignals diskret verteilt sind, er also zur Erzeugung eines Linienspektrums vorgesehen ist. Die Verwendung eines Linienspektrums ermöglicht es, die im Generatorsignal enthaltene Energie auf die zur Auswertung vorgesehenen Frequenzen zu bündeln.

Besonders bevorzugt ist der Signalgenerator so ausgebildet, dass die besagten diskreten Frequenzen im Spektrum des Generatorsignals äquidistant verteilt sind. Ein solches äquidistantes Linienspektrum kann in einfacher Weise erzeugt werden, indem sinusförmige Signale überlagert werden, deren Frequenz ein ganzzahliges Vielfaches einer Grundfrequenz ist.

Dabei ist vorteilhafterweise vorgesehen, dass der Signalgenerator so ausgebildet ist, dass der Abstand benachbarter Frequenzen weniger als das 1,5-fache, bevorzugt weniger als das 1-fache, besonders bevorzugt weniger als das 0,75-fache, der minimalen Bandbreite des Mikrowellenresonators beträgt. Die minimale Bandbreite des Mikrowellenresonators ist durch den minimalen Wert des Quotienten aus Resonanzfrequenz und Güte bei jeweils gleicher Frequenz definiert. Durch die vorgeschlagenen geringen Abstände der Spektrallinien ist sichergestellt, dass unabhängig vom Füllgrad des Messraumes auswertbare Frequenzen zur Verfügung stehen, welche in der Nähe der jeweiligen Resonanzfrequenz liegen. Hierdurch kann die erforderliche Genauigkeit der Messung in jedem Fall garantiert werden.

In einer besonders vorteilhaften Ausführungsform ist der Signalgenerator so ausgebildet, dass wenigstens die zur Auswertung vorgesehenen besagten Frequenzen im Spektrum des Generatorsignals gleiche Amplituden aufweisen.

Hierdurch vereinfacht sich die Auswertung des Ausgangssignals beträchtlich.

In einer vorteilhaften Ausführungsform ist die Auswerteschaltung zur Erzeugung eines mit der Resonanzfrequenz des Mikrowellenresonators korrespondierenden Frequenzsignals und/oder zur Erzeugung eines mit der Güte des Mikrowellenresonators korrespondierenden Gütesignals ausgebildet. Das Frequenzsignal und/oder das Gütesignal kann dann in bekannter Weise auch mittels bestehender Vorrichtungen ausgewertet werden.

Bevorzugt ist die Auswerteschaltung zur Bestimmung der Amplitude von wenigstens drei der besagten Frequenzen im Spektrum des Ausgangssignals ausgebildet. Aus drei verschiedenen Amplitudenwerten kann die Amplitudenübertragungsfunktion eindeutig bestimmt werden. Die Messung von Phasenwerten ist dann nicht erforderlich.

In einer anderen vorteilhaften Ausführungsform ist die Auswerteschaltung zur Bestimmung der Amplitude und der Phase von wenigstens zwei der besagten Frequenzen im Spektrum des Ausgangssignals ausgebildet. Auch in diesem Fall ist die Übertragungsfunktion des Mikrowellenresonators eindeutig bestimmbar.

Wenn die Phase von wenigstens drei der besagten Frequenzen im Spektrum ermittelt wird, so kann auf die Bestimmung von Amplitudenwerten verzichtet werden.

Wenn die Anzahl der ausgewerteten Frequenzen größer als erforderlich ist, so ergibt sich für die Resonanzfrequenz und/oder die Güte in vielen Fällen keine exakte Lösung. In diesem Fall ist es vorteilhaft, wenn die Auswerteschaltung zur Bestimmung der Resonanzfrequenz und/oder der Güte des Mikrowellenresonators unter Anwendung eines Fehlerminimierungsverfahrens ausgebildet ist. Geeignete Fehlerminimierungsverfahren sind beispielsweise die Methode des (evtl. gewichteten) minimalen Fehlerbetrages oder des (evtl. gewichteten) minimalen Fehlerquadrates. Die Anwendung derartiger Fehlerminimierungsverfahren erlaubt die Berücksichtigung beliebig vieler Frequenzen bei der Ermittlung der Resonanzfrequenz und/oder der Güte des Mikrowellenoszillators.

Um die Genauigkeit der Messungen zu erhöhen, ist es vorteilhaft, wenn die Auswerteschaltung zur Bestimmung der Amplitude und/oder der Phase einer innerhalb der Bandbreite des Mikrowellenresonators gelegenen besagten Frequenz im Spektrum des Ausgangssignals ausgebildet ist.

Ebenso dient es der Erhöhung der Genauigkeit der Messungen, wenn die Auswerteschaltung zur Bestimmung der Amplitude und/oder der Phase wenigstens einer oberhalb der Resonanzfrequenz des Mikrowellenresonators gelegenen besagten Frequenz und wenigstens einer unterhalb der Resonanzfrequenz des Mikrowellenresonators gelegenen besagten Frequenz im Spektrum des Ausgangssignals ausgebildet ist.

Besonders vorteilhaft ist es, wenn die Auswerteschaltung zur Berücksichtigung ungleicher Amplituden der zur Auswertung vorgesehenen besagten Frequenzen im Spektrum des Generatorsignals ausgebildet ist. Die Berücksichtigung ungleicher Amplituden kann derart erfolgen, dass die Amplitude einer bestimmten besagten Frequenz im Spektrum des Ausgangssignals mit der Amplitude derselben Frequenz im Spektrum des Generatorsignals verglichen wird, wobei letztere Information in dem der Auswerteschaltung zugeführten Synchronsignal enthalten sein kann. Erfindungsgemäß umfasst die Auswerteschaltung eine Analyseschaltung zur Spektralanalyse eines durch eine Abtastschaltung erzeugten Abtastsignals. Die Abtastschaltung ist dabei so ausgebildet, dass das Abtastsignal mit dem Ausgangssignal korrespondiert. In dem mittels der Spektralanalyse erzeugten Spektrum des Abtastsignals sind folglich Informationen über die Amplituden und/oder Phasen der im Ausgangssignal enthaltenen Frequenzen enthalten. Damit können aus der Spektralanalyse des Abtastsignals Resonanzfrequenz und/oder Güte des Mikrowellenresonators ermittelt werden.

In einer vorteilhaften Ausführungsform ist die Abtastschaltung zum Abtasten des Ausgangssignals selbst ausgebildet. Es ergibt sich somit eine einfache Anordnung.

In einer weiteren vorteilhaften Ausführungsform umfasst die Auswerteschaltung eine Transponierschaltung zum Transponieren des Ausgangssignals in ein niederfrequentes Basisband. Das durch die Transponierschaltung erzeugte transponierte Ausgangssignal wird dann durch die hierzu ausgebildete Abtastschaltung abgetastet. Das Transponieren des Ausgangssignals erfolgt in bekannter Weise durch Mischen mit einem harmonischen Signal. Durch das Transponieren des Ausgangssignals in das niederfrequente Basisband vereinfacht sich die weitere Verarbeitung des Signals.

Vorteilhafterweise erzeugt die Abtastschaltung ein digitales Abtastsignal, wobei die Analyseschaltung als digitale Analyseschaltung zur Analyse des digitalen Abtastsignals ausgebildet ist. Digitale Analyseschaltung stehen kostengünstig zur Verfügung.

Vorteilhafterweise ist die Analyseschaltung zur Spektralanalyse des Abtastsignals auf der Basis der schnellen Fourier-Transformation und/oder auf der Basis der Prony'schen Methode ausgebildet. Bei den genannten Verfahren handelt es sich um Näherungsverfahren, welche es erlauben, das gesuchte Spektrum mit hinreichender Genauigkeit schnell und einfach zu bestimmen. Vorteilhafterweise ist der Mikrowellenresonator als Zweitor-Resonator ausgebildet. Er weist dann eine Einkoppelanordnung, über die das Generatorsignal in den Mikrowellenresonator eingekoppelt ist und eine Auskoppelanordnung, über die das Ausgangssignal aus dem Mikrowellenresonator ausgekoppelt ist, auf. Mit einem Zweitor-Resonator können in einfacher Weise Durchgangsmessungen durchgeführt werden.

In einer weiteren Ausführungsform ist der Mikrowellenresonator als Eintor-Resonator ausgebildet. Hier weist der Mikrowellenresonator eine Koppelanordnung auf, über welche das Generatorsignal in den Mikrowellenresonator eingekoppelt und ein reflektiertes Signal als Ausgangssignal ausgekoppelt ist. Ein derartiger Mikrowellenresonator zur Durchführung von Reflektionsmessungen ist verhältnismäßig einfach im mechanischen Aufbau.

In einer vorteilhaften Ausführungsform mit einem Eintor-Resonator ist der Signalgenerator so ausgebildet, dass das Generatorsignal ein eingeprägter Strom ist und dass die Auswerteschaltung zur Auswertung der als reflektiertes Signal an der Koppelanordnung auftretenden Spannung ausgebildet ist.

In einer ebenso vorteilhaften Ausführungsform mit einem Eintor-Resonator ist der Signalgenerator so ausgebildet, dass das Generatorsignal eine eingeprägte Spannung ist und dass die Auswerteschaltung zur Auswertung des als reflektiertes Ausgangssignal in der Koppelanordnung fließenden Stromes ausgebildet ist.

Im Falle der Ein- und Auskopplung der Signale über Wellenleiter (z.B. Hohlleiter) kann der Signalgenerator so ausgebildet sein, dass das Generatorsignal eine eingeprägte Welle ist und dass die Auswerteschaltung zur Auswertung der Amplitude und/oder Phase des als reflektierte Welle an der Koppelanordnung auftretenden Ausgangssignals ausgebildet ist.

In vorteilhafter Weiterbildung der Erfindung ist eine Signalauswertestufe vorgesehen, welche aus dem Frequenzsignal und/oder aus dem Gütesignal Messwerte der längenspezifischen Masse und/oder Messwerte der Feuchtigkeit des strangförmigen laufenden Fasergemenges erzeugt. Eine derart ausgebildete Vorrichtung kann unmittelbar bei einer herkömmlichen Textilmaschine eingesetzt werden.

Vorteilhafterweise ist wenigstens ein FPGA vorgesehen. Bevorzugt ist vorgesehen, die Signalauswertestufe mittels eines entsprechend eingerichteten FPGAs zu realisieren. Es ist jedoch auch möglich, den Signalgenerator und/oder die Auswerteschaltung durch jeweils einen entsprechend modifizierten FPGA auszubilden. Vorteilhafterweise ist ein einzelner FPGA so eingerichtet, dass er die Funktion mehrerer oder aller der genannten Baugruppen übernimmt.

Bei einem FPGA (Abkürzung für "Field Programmable Gate Array") handelt es sich um einen modifizierbaren Logikbaustein, dessen Funktion durch spezifische Konfiguration interner Strukturen festlegbar ist. Daher können mit ein und demselben FPGA nahezu beliebige Schaltungen realisiert werden. Hierdurch ergibt sich ein Kostenvorteil im Vergleich zur Verwendung eine Anwendungsspezifischen Integrierten Schaltung (ASIC). Im Gegensatz zu einem Mikroprozessor, dessen Funktion durch ein sequentiell abzuarbeitendes Programm festgelegt werden muss, bietet der FPGA die Möglichkeit der parallelen Informationsverarbeitung, was zu einer höheren Verarbeitungsgeschwindigkeit führt.

Bevorzugt sind der Mikrowellenresonator und zumindest Teile der daran angeschlossenen Schaltung, insbesondere die Signalauswertestufe, der Signalgenerator und/oder die Auswerteschaltung in einem gemeinsamen Gehäuse angeordnet. Hierdurch kann der in einem textilen Umfeld erforderliche Schutz der Gesamtvorrichtung gegen Schmutz, Faserflug, Temperaturschwankungen, mechanische Einwirkungen usw. in einfacher Weise bewerkstelligt werden.

Mit Vorteil weist die Vorrichtung eine Kommunikationsschnittstelle mit einem definierten Protokoll zum Anbinden der Vorrichtung an eine Kommunikationsverbindung einer Textilmaschine auf. Moderne Textilmaschinen weisen eine Vielzahl steuerungsrelevanter Baugruppen auf. Dabei sind Kommunikationsverbindungen zum Verbinden dieser Baugruppen vorgesehen. Wenn also die erfindungsgemäße Vorrichtung eine nach einem entsprechenden Protokoll arbeitende Kommunikationsschnittstelle aufweist, so ermöglicht dies eine einfache Montage der Vorrichtung bei der Produktion einer Textilmaschine oder bei einer nachträglichen Umrüstung einer solchen. Bei Verwendung einer Bus-Schnittstelle kann der Verkabelungsaufwand minimiert werden. Besonders geeignet sind beispielsweise CAN-Bus-Schnittstellen.

Eine erfindungsgemäße Textilmaschine ist dadurch gekennzeichnet, dass wenigstens eine erfindungsgemäße Vorrichtung vorgesehen ist. Es ergeben sich die beschriebenen Vorteile.

Weitere Vorteile der Erfindung sind in den nachfolgenden Ausführungsbeispielen beschrieben. Es zeigen:

**Figur 1**     eine Strecke als Beispiel für eine Textilmaschine nach dem Stand der Technik,

**Figur 2**     eine erfindungsgemäße Strecke,

**Figur 3**     eine erfindungsgemäße Vorrichtung mit einem Zweitor- Mikrowellenresonator,

**Figur 4**     ein Ersatzschaltbild der Vorrichtung nach Figur 3,

**Figur 5a**     ein äquidistantes Linienspektrum eines Generatorsignals,

**Figur 5b**     das zur Figur 5a gehörige Generatorsignal,

**Figur 6a**     ein bandbegrenztes äquidistantes Linienspektrum eines Gene- ratorsignals,

**Figur 6b**     das zur Figur 6a gehörige Generatorsignal,

**Figur 7**     eine Skizze zur Amplituden- und Phasenübertragung eines Mikrowellenresonators sowie eines Spektrums eines Genera- torsignals,

**Figur 8** eine Darstellung des Betrages, des Realteiles und des Imagi- närteiles einer Übertragungsfunktion eines Mikrowellenresona- tors,

**Figur 9** den Betrag des Spektrums eines Ausgangssignals,

**Figur 10** den Realteil des Spektrums des Ausgangssignals,

**Figur 11** den Imaginärteil den Spektrum des Ausgangssignals,

**Figur 12** eine erfindungsgemäße Vorrichtung mit einem Eintor- Mikrowellenresonator,

**Figur 13** ein Ersatzschaltbild der Vorrichtung nach Figur 12,

**Figur 14** eine weitere erfindungsgemäße Vorrichtung mit einem Eintor- Mikrowellenresonator,

**Figur 15** ein Ersatzschaltbild der Vorrichtung nach Figur 14,

**Figur 16** eine weitere erfindungsgemäße Vorrichtung mit einem Zweitor- Mikrowellenresonator,

**Figur 17** ein Ersatzschaltbild der Vorrichtung nach Figur 17,

**Figur 18** ein weiteres Ausführungsbeispiel einer erfindungsgemäßen Vorrichtung,

**Figur 19** ein Ersatzschaltbild der Vorrichtung nach Figur 18,

**Figur 20** das in das Basisband transponiertes Ausgangssignal und

**Figur 21** eine perspektivische Darstellung eines Mikrowellenresonators.

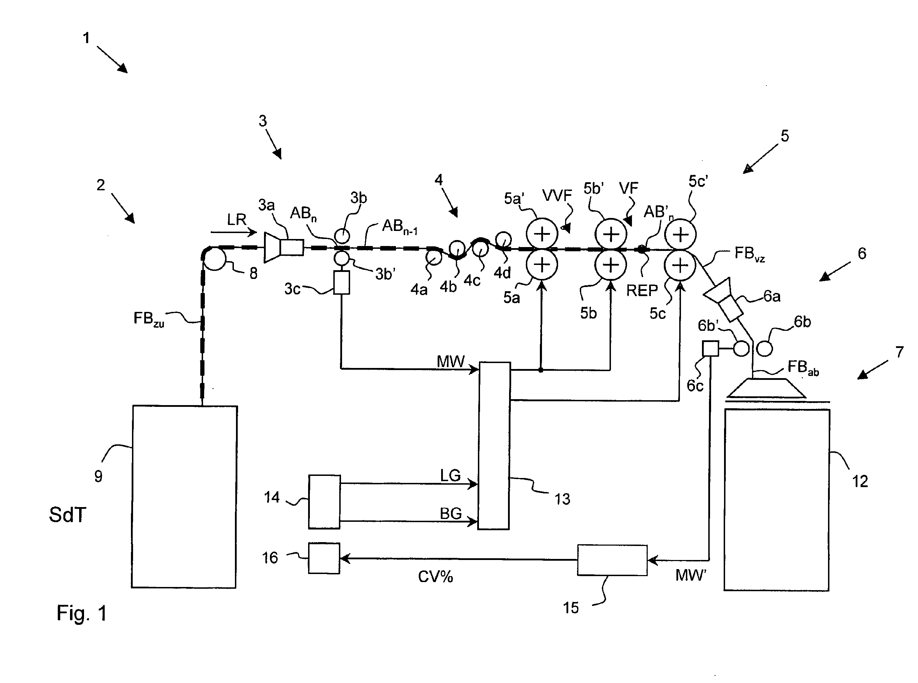

Figur 1 zeigt eine nach dem Stand der Technik ausgebildete Strecke 1, als Beispiel für eine Textilmaschine 1. Das der Strecke 1 vorgelegte Fasergemenge FB wird in Laufrichtung LR über eine Bandzuführung 2, eine Einlaufsensoreinheit 3, eine Umlenkeinheit 4, ein Streckwerk 5, eine Auslaufführung 6 und über eine Bandablage 7 geführt.
Die nur skizzenhaft dargestellte Bandzuführung 2 weist ein Umlenkrohr 8 auf, welches so angeordnet ist, dass das vorgelegte Fasergemenge $FB_{zu}$ in Form eines Faserbandes $FB_{zu}$ aus einer der Strecke 1 beigestellten Spinnkanne 9 entnommen werden kann. Die Bandzuführung 2 könnte jedoch auch so ausgebildet sein, dass sie ein vorgelegtes Faserband $FB_{zu}$ direkt von einer laufenden Karde übernehmen kann. Ebenfalls, und das ist in der Praxis der häufigste Fall, könnte die Bandzuführung 2 zur gleichzeitigen Entnahme mehrerer vorgelegter Faserbänder $FB_{zu}$ aus verschiedenen beigestellten Spinnkannen 9 ausgebildet sein. Wenn im Folgenden von Faserband $FB_{zu}$ gesprochen wird, soll dadurch nicht ausgeschlossen werden, dass auch mehrere Faserbändem $FB_{zu}$ gemeint sind.
Das vorgelegte Faserband $FB_{zu}$ wird von der Bandzuführung 2 zu der Einzugseinheit 3 transportiert. Diese umfasst einen Einlauftrichter 3a und ein angetriebenes Tastwalzenpaar 3b, 3b', welches aus einer ortsfest gelagerten Walze 3b sowie einer beweglich gelagerten und belasteten Walze 3b' besteht.
Das Tastwalzenpaar 3b, 3b' dient einerseits dem Hindurchziehen des Faserbandes $FB_{zu}$ durch den Einlauftrichter 3a, um es so zu komprimieren und andererseits der abschnittsweisen Erfassung der längenspezifischen Masse des hindurchgeführten Faserbandes $FB_{zu}$. Zu letzterem ist ein Wegaufnehmer 3c zur Erfassung der Bewegung der beweglichen Walze 3b' vorgesehen. Letztlich wird somit der Querschnitt des Faserbandes $FB_{zu}$ erfasst und daraus auf die längenspezifische Masse des Faserbandes $FB_{zu}$ geschlossen. Im Einlaufbereich, also stromaufwärts des Streckwerks 5, erfolgt die Erfassung der längenspezifischen Masse des hindurchgeführten Fasergemenges $FB_{zu}$. Also durch mechanische Abtastung. Die einzelnen vermessenen Abschnitte AB weisen dabei eine Länge von einigen Millimetern auf. Für jeden vermessenen Abschnitt AB wird durch den Wegaufnehmer 3c ein Messwert MW erzeugt.
Die Umlenkeinheit 4 mit den Bandumlenkstäben 4a, 4b, 4c und 4d dient dazu, das von der Einzugseinheit 3 kommende Faserband $FB_{zu}$ quer zur Laufrichtung gleichmäßig auszubreiten und dabei die im Bereich der Einzugseinheit 3 erfolgte Pressung des Faserbandes $FB_{zu}$ aufzulösen.
Beim Transport des Faserbandes $FB_{zu}$ von der Einzugseinheit 3 zum Streckwerk 5 wird das Faserband $FB_{zu}$ einer Einzugsspannung unterworfen, welche durch eine unterschiedliche Umfangsgeschwindigkeit des Tastwalzenpaares 3a, 3a' und des Einzugswalzenpaares 5a, 5a' bewirkt wird.
Das Streckwerk 5 umfasst ein Eingangswalzenpaar 5a, 5a' sowie ein Mittelwalzenpaar 5b, 5b' und ein Lieferwalzenpaar 5c, 5c'. Die Walzenpaare 5a, 5a'; 5b, 5b'; 5c, 5c'; sind derart angetrieben, dass die Drehzahl von Walzenpaar zu Walzenpaar in Laufrichtung zu nimmt. Hierdurch wird das Faserband $FB_{zu}$ sowohl im Vorverzugsfeld WF, welches zwischen dem Eingangswalzenpaar 5a, 5a' und dem Mittelwalzenpaar 5b, 5b' gebildet ist, als auch im Hauptverzugsfeld VF, welches zwischen dem Mittelwalzenpaar 5b, 5b' und dem Lieferwalzenpaar 5c, 5c' gebildet ist, verzogen.

Die Unterwalzen 5a, 5d, 5c des Streckwerkes 5 sind ortsfest angeordnet, hingegen sind die Oberwalzen 5a', 5b' 5c' beweglich gelagert und werden mittels nicht gezeigter Belastungsmittel gegen die Unterwalzen 5a, 5b, 5c gedrückt, so dass sich eine sichere Klemmung des Faserbandes $FB_{zu}$ ergibt.

Die Auslaufführung 6 umfasst einen Auslaufrichter 6a sowie ein angetriebenes Abzugswalzenpaar 6b, 6b', von denen eine Walze 6b ortsfest gelagert und die andere Walze 6b' beweglich gelagert ist. Der Auslauftrichter 6a bewirkt eine Komprimierung des verzogenen Faserbandes $FB_{vz}$ so dass ein kompaktes Faserband $FB_{ab}$ entsteht.

Die Abzugswalzen 6b, 6b' dienen zunächst dem Abziehen des Faserbandes $FB_{ab}$ aus dem Auslauftrichter 6a sowie der weiteren Kompaktierung des Faserbandes $FB_{ab}$. Darüber hinaus ist der beweglich gelagerten Walze 6b' ein weiterer Wegaufnehmer 6c zur Erzeugung von Messwerten MW', welche mit der längenspezifischen Masse des abgeführten Faserbandes $FB_{ab}$ korrespondiert, zugeordnet. Damit kommt bei herkömmlichen Strecken 1 auch im Auslaufbereich, also stromabwärts des Streckwerks 5, das Prinzip der mechanischen Abtastung zur Bestimmung der längenspezifischen Masse des abgeführten Fasergemenges $FB_{ab}$ zur Anwendung.

Die Bandablage 7, welche hier nicht im Detail erläutert wird, bewirkt die geordnete Ablage des mittels der Strecke 1 erzeugten Faserbandes $FB_{ab}$ in eine Spinnkanne 12.

Das Streckwerk 5 ist über eine Steuerungsvorrichtung 13 steuerbar. Dabei ist der Steuerungsvorrichtung 13 eine Bedieneinheit 14 zugeordnet, welche es einem Bediener ermöglicht, Einstellwerte vorzugeben, welche dann als Eingangsgröße an die Steuerungsvorrichtung 13 übermittelt werden. Derartige durch den Bediener vorgebbare Eingangsgrößen sind beispielsweise die gewünschte Liefergeschwindigkeit LG und das gewünschte Bandgewicht BG erzeugten Faserbandes $FB_{ab}$. Die Liefergeschwindigkeit LG ist dabei jene Geschwindigkeit, mit der die verzogenen Faserbänder FB das Streckwerk 5 verlassen. Das Bandgewicht BG hingegen beschreibt die durchschnittliche längenspezifische Masse des von der Strecke 1 abgegebenen Faserbandes $FB_{ab}$. Weitere Eingangsgröße der Steuerungsvorrichtung 13 ist der aktuelle Messwert MW, der von der Einlaufsensoreinheit 3 automatisch zur Steuerungsvorrichtung 13 übertragen wird. Dargestellt sind lediglich ausgewählte Eingangsgrößen der Steuerungsvorrichtung 13. In der Praxis werden der Steuerungsvorrichtung 13 weitere Eingangsgrößen zugeführt.

Die Steuerungsvorrichtung 13 ist so ausgebildet, dass sie in Abhängigkeit von ihren Eingangsgrößen die Drehzahl des Eingangswalzenpaares 5a, 5a', die Drehzahl des Mittelwalzenpaares 5b, 5b' und die Drehzahl des Lieferwalzenaares 5c, 5c' durch Einwirkung auf nicht gezeigte Antriebsmittel steuert. Im unregulierten Betrieb wird dabei für das Vorverzugsfeld VVF ein fester Vorverzug und für das Hauptverzugsfeld VF ein fester Hauptverzug festgelegt.

Im regulierten Betrieb hingegen sind Verzugsänderungen, auch Regeleingriffe genannt, vorgesehen, um das dem Streckwerk 5 zugeführte Faserband $FB_{zu}$ zu vergleichmäßigen. Derartige Regeleingriffe erfolgen auf der Basis der Messungen des Wegaufnehmers 3c. Eine Steuerung, bei welcher der Messort vor dem Streckwerk 5 liegt nennt man üblicherweise Open-Loop-Steuerung. Bei einer derartigen Steuerung ist die Laufstrecke bzw. die Laufzeit eines Abschnittes AB der zugeführten Faserband $FB_{zu}$ bis zu dem Punkt REP, an dem der Regeleingriff erfolgen soll, zu berücksichtigen. Laufstrecke und Laufzeit sind über die Einzugsgeschwindigkeit des Streckwerks miteinander verknüpft.

Das dem Verzugsfeld VF zugeführte Fasergemenge $FB_{zu}$ besteht aus nacheinander angeordneten Abschnit-ten. Durch das Bezugszeichen $AB_n$ ist der Abschnitt, der im dargestellten Moment durch die Sensoreinrichtung 3c vermessen wird, bezeichnet. Stromabwärts des Abschnitts $AB_n$ liegt der Abschnitt $AB_{n-1}$. Aus Vereinfachungsgründen sind die weiteren Abschnitte nicht durch Bezugszeichen benannt. Für jeden der Abschnitte wird wenigstens ein Messwert MW ermittelt, der mit der längenspezifischen Masse des jeweiligen Abschnitts AB korrespondiert und der an die Steuerungseinrichtung 13 übermittelt wird.

Wenn der vermessene Abschnitt $AB_n$ den Regeleinsatzpunkt REP, also die mit $AB'_n$ bezeichnete Position erreicht, wird durch die Steuerungseinrichtung 13 ein entsprechender Regeleingriff veranlasst. Weist der Abschnitt $AB_n$ beispielsweise eine über dem Durchschnitt liegende längenspezifische Masse auf, so wird eine Erhöhung des Verzugs zur Vergleichmäßigung des Fasergemenges FB eingeleitet.

Wesentlich für die Qualität der erzeugten Faserbandes $FB_{ab}$ ist insbesondere die Genauigkeit der Messwerte MW, welche die längenspezifische Masse des zugeführten Faserbandes $FB_{zu}$ abbilden. Dabei ist die Qualität um so höher, je gleichmäßiger das erzeugte Faserband $FB_{ab}$ ist.

Zur Überprüfung der Qualität der erzeugten Faserbandes $FB_{ab}$ ist es aus dem Stand der Technik bekannt, mittels einer Auswerteeinheit 15 aus den Messwerten MW' des Messtrichters 6a einen Variationskoeffizient zu errechnen, der die prozentuale Bandungleichmäßigkeit für eine bestimmte Bezugslänge des Faserbandes $FB_{ab}$ angibt. Die Bezugslänge wird auch Schnittlänge, der Variationskoeffizient auch CV%-Wert genannt. In der Praxis liegen die verwendeten Schnittlängen im Bereich zwischen einigen Zentimetern und einigen Metern. Verlässliche CV%-Werte können allerdings nur erhalten werden, wenn die Messwerte MW' eine hohe Genauigkeit aufweisen. Die ermittelten CV%-Werte werden beispielsweise an einer Anzeige 16 dargestellt oder zur späteren Auswertung abgespeichert.

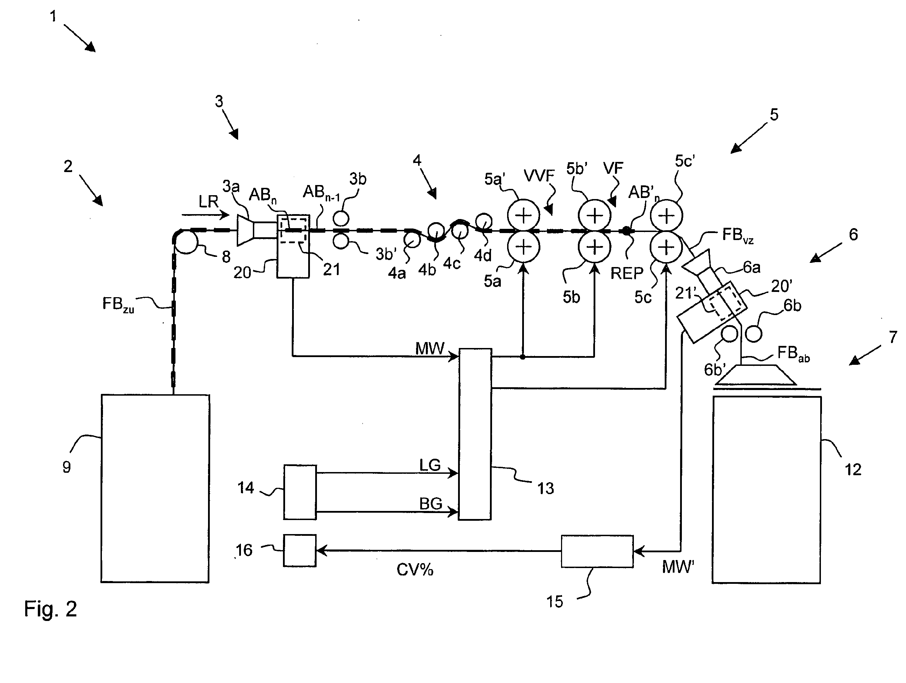

Figur 2 zeigt eine erfindungsgemäße Strecke 1 als Beispiel für eine erfindungsgemäße Textilmaschine 1. Sie zeichnet sich dadurch aus, dass eine erste erfindungsgemäße Vorrichtung 20 im Bereich der Einzugseinheit 3 und eine zweite erfindungsgemäße Vorrichtung 20' im Bereich der Auslaufführung 6 vorgesehen ist. Die erste erfindungsgemäße Vorrichtung 20 ersetzt den Wegaufnehmer 3c der in der Figur 1 dargestellten Strecke 1. Die Vorrichtung 20 dient dabei ebenfalls der Erfassung der längenspezifischen Masse der Abschnitte AB des dem Streckwerk 5 zugeführten Faserbandes $FB_{zu}$ sowie der Erzeugung von Messwerten, welche diese Informationen umfassen.

Die Messwerte MW werden in der bekannten Weise zur Steuerung der Strecke 1 verwendet. Die Erfassung der längenspezifischen Masse erfolgt dabei berührungslos unter Verwendung von Mikrowellen. Wegen der fehlenden Massenträgheit kann somit die Arbeitsgeschwindigkeit der Strecke prinzipiell erhöht werden. Die Erfassung der längenspezifischen Masse des Faserbandes FB erfolgt auf der Basis einer Auswertung der dielektrischen Eigenschaften des Messraumes 22 der erfindungsgemäßen Vorrichtung 20. Die mittels der Vorrichtung 20 erzeugten Messwerte MW bilden die längenspezifische Trockenmasse des Fasergemenges $FB_{zu}$ ab, so dass Fehler bei der Steuerung der Textilmaschine 1 durch Veränderungen des Feuchtegehaltes des Faserbandes $FB_{zu}$ vermieden sind. Da die Messzeit der erfindungsgemäßen Vorrichtung 20 gegenüber bekannten Mikrowellenmessvorrichtungen verringert ist, ergibt sich eine hohe Genauigkeit der Messwerte MW auch dann, wenn das Fasergemenge $FB_{zu}$ mit hoher Geschwindigkeit bewegt wird. Insgesamt wird eine Mittelung der längenspezifischen Masse mehrerer aufeinanderfolgender Abschnitte AB vermieden. Jeder Messwert MW repräsentiert genau einen Abschnitt AB.

Die weitere erfindungsgemäße Vorrichtung 20' erfasst abschnittsweise die längenspezifische Masse des abzulegenden Fasergemenges $FB_{ab}$. Die mit der längenspezifischen Masse korrespondierenden Messwerte MW' werden in bekannter Weise der Auswerteeinheit 15 zugeführt und zur Erzeugung von qualitätskennzeichnenden Größen herangezogen. Die Messwerte MW' werden auf der Basis der dielektrischen Eigenschaften des Messraumes 21' erzeugt. Die Erfassung dieser dielektrischen Eigenschaften erfolgt analog zur Funktionsweise der erfindungsgemäßen Vorrichtung 20. Es ergeben sich daher auch dieselben Vorteile. Zu bemerken ist allerdings, dass die Anordnung einer erfindungsgemäßen Vorrichtung 20' gerade im Bereich der Auslaufführung 6 von Vorteil ist, da üblicherweise die Geschwindigkeit des hier zu vermessenden Fasergemenges $FB_{ab}$ höher ist.

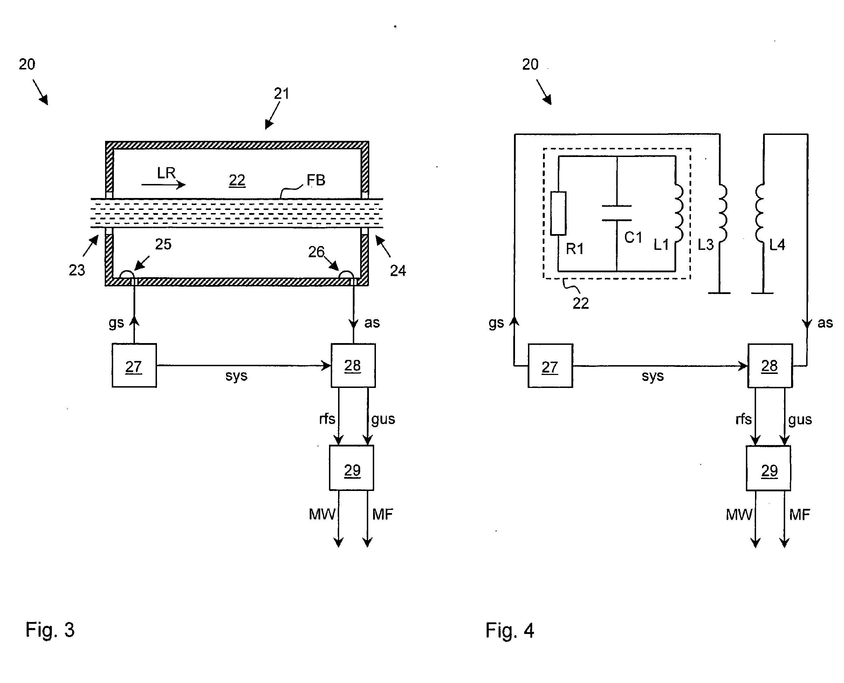

Figur 3 zeigt ein erstes Ausführungsbeispiel einer erfindungsgemäßen Vorrichtung 20. Sie umfasst einen Mikrowellenresonator 21 mit einem Messraum 22. Durch eine Eingangsöffnung 23 und durch eine Ausgangsöffnung 24 kann das zu vermessende Faserband FB in Laufrichtung LR durch den Messraum 22 kontinuierlich hindurchgefördert werden. Der Mikrowellenresonator 21 weist weiterhin eine Einkoppelanordnung 25 und eine Auskoppelanordnung 26 auf. Er ist somit als Zweitor-Mikrowellenresonator ausgebildet.

Erfindungsgemäß ist ein Signalgenerator 27 vorgesehen, dessen Generatorsignal gs mittels der Einkoppelanordnung 25 in den Messraum 22 des Mikrowellenresonators 21 eingekoppelt ist. Weiterhin vorgesehen ist eine Auswerteschaltung 28, der ein über die Auskoppelanordnung 26 aus dem Messraum 22 ausgekoppeltes Ausgangssignal as zugeführt ist. Die Auswerteschaltung 28 ist zur Erzeugung eines mit der Resonanzfrequenz des Mikrowellenresonators 21 korrespondierenden Frequenzsignals rfs und zur Erzeugung eines mit der Güte des Mikrowellenresonators 21 korrespondierenden Gütesignals gus ausgebildet. Weiterhin ist eine Signalauswertestufe 29 vorgesehen, welche aus dem Frequenzsignal rfs und aus dem Gütesignal gus Messwerte MW der längenspezifischen Masse und Messwerte MF der Feuchtigkeit strangförmigen laufenden Fasergemenges erzeugt.

Figur 4 zeigt ein Ersatzschaltbild der anhand der Figur 3 erläuterten Vorrichtung 20. Die elektrischen Eigenschaften des Mikrowellenresonators 21 können durch den Widerstand R1, die Kapazität C1 und die Induktivität L1, welche parallel geschaltet sind, dargestellt werden. Der Mikrowellenresonator stellt also in elektrischer Hinsicht einen Parallelschwingkreis R1, C1, L1 dar. Sowohl die Masse des Trockenanteils als auch die Masse des Feuchtanteils des im Messraum 22 befindlichen Fasergemenges FB beeinflussen den Wert des Widerstandes R1 und den Wert der Kapazität C1. Hierdurch wird sowohl die Güte als auch die Resonanzfrequenz des Parallelschwingkreises R1, C1, L1 verändert. Da jedoch der Einfluss des Trockenanteils des Faserbandes auf die Kenngrößen des Parallelschwingkreises R1, C1, L1 anderen Gesetzmäßigkeiten folgt als der Einfluss des Feuchtanteils des Faserbandes FB, kann aus der Kenntnis der Güte und der Resonanzfrequenz des Parallelschwingkreises R1, C1, L1 sowohl auf die längenspezifische Masse des Feuchtanteils als auch auf die längenspezifische Masse des Trockenanteils des im Messraum 22 befindlichen Fasergemenges FB geschlossen werden. Die in der Figur 3 gezeigte Einkoppelanordnung 25 und die Auskoppelanordnung 26 sind in der Figur 4 durch die Induktivität L3 bzw. durch die Induktivität L4 repräsentiert.

Kern der Erfindung ist es, dass das Spektrum des Generatorsignals gs mehrere Frequenzen umfasst, wobei für eine Mehrzahl der im Spektrum des Generatorsignals gs enthaltenen Frequenzen die Amplitude und/oder die Phase im Spektrum des Ausgangssignals as simultan bestimmt wird. Durch die Auswertung des Ausgangssignals as kann bei bekanntem Generatorsignal gs auf die Resonanzfrequenz und auf die Güte des Mikrowellenresonators 21 geschlossen werden. Daher wird der Auswerteschaltung 28 vom Generator 27 ein Synchronsignal sys zugeführt, welches mit dem Generatorsignal gs korrespondiert.

Jede im Spektrum des Generatorsignals gs enthaltene Frequenz ist auch in dem Spektrum des Ausgangssignals as enthalten. Wird nun für eine hinreichend große Anzahl von Frequenzen im Spektrum des Ausgangssignals as die jeweilige Amplitude bestimmt, so kann die Amplitudenübertragungsfunktion des Mikrowellenresonators, auch Resonanzkurve genannt, eindeutig bestimmt werden. Ebenso kann durch eine Auswertung der Phasenlage einer hinreichend großen Anzahl von Frequenzen im Ausgangssignal as die Phasenübertragungsfunktion bestimmt werden. Sowohl aus der Amplitudenübertragungsfunktion als auch aus der Phasenübertragungsfunktion kann dann die Resonanzfrequenz und/oder die Güte des Mikrowellenresonators bestimmt werden. Zur Erhöhung der Genauigkeit der ermittelten Resonanzfrequenz bzw. Güte ist es jedoch in vielen Fällen sinnvoll, wenn sowohl die Amplitudenübertragungsfunktion als auch die Phasenübertragungsfunktion ermittelt und ausgewertet werden.

Im Ergebnis kann mit der dargestellten Vorrichtung 20 ein mit der Resonanzfrequenz des Mikrowellenresonators 21 korrespondierendes Signal rfs und ein mit der Güte des Mikrowellenresonators 21 korrespondierendes Signal gus erzeugt werden, ohne dass es wie bei bekannten Mikrowellenvorrichtungen der schrittweisen Aufnahme einer Resonanzkurve bedarf.

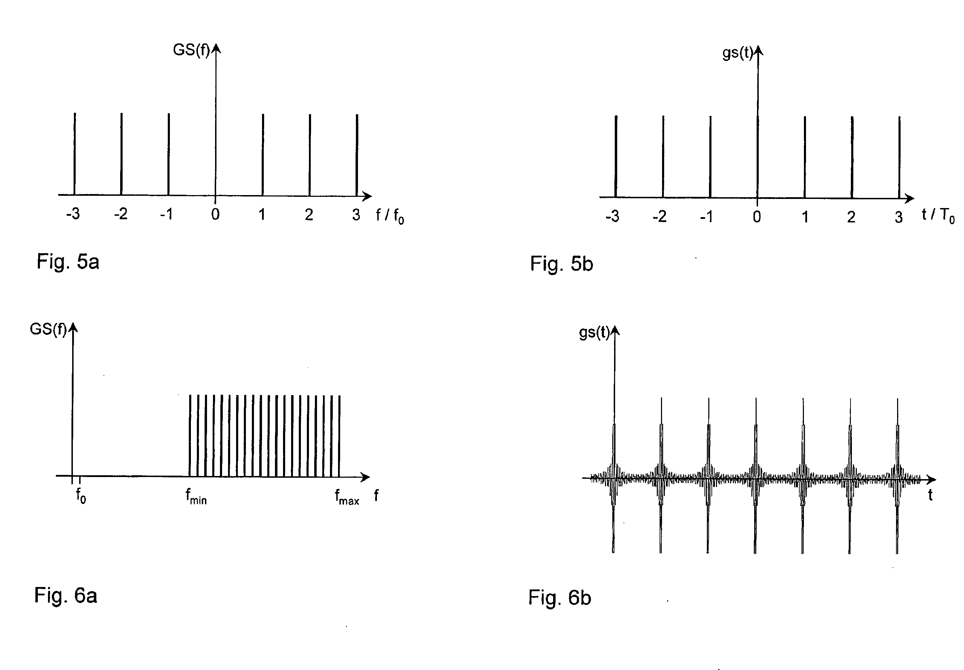

Figur 5 zeigt ein Spektrum GS eines Generatorsignals gs, welches mehrere Frequenzen umfasst. Bei dem dargestellten Spektrum GS handelt es sich um ein Linienspektrum mit unendlicher Bandbreite. Bei den im Spektrum GS enthaltenen Frequenzen handelt es sich um ganzzahlige Vielfache einer Grundfrequenz $f_0$. Da benachbarte Frequenzen denselben Abstand aufweisen, wird ein derartiges Spektrum GS als äquidistant bezeichnet. Mathematische Überlegungen zeigen, dass das in der Figur 5a gezeigte Spektrum GS das Spektrum eines in Figur 5b dargestellten Generatorsignals gs ist, welches aus einer periodischen Folge von Nadelimpulsen besteht.

Grundsätzlich ist das in der Figur 5b dargestellte Generatorsignal gs zur Verwendung bei einer erfindungsgemäßen Vorrichtung geeignet. Allerdings bewirkt die unendliche Bandbreite des zugehörigen Spektrums GS, das eine Vielzahl von Frequenzen zwar vorhanden, aber für die weitere Auswertung ohne Bedeutung ist.

Bevorzugt wird daher ein Generatorsignal gs verwendet, dessen Spektrum GS ein bandbegrenztes äquidistantes Linienspektrum ist. Ein derartiges bandbegrenztes äquidistantes Linienspektrum GS ist beispielhaft in Figur 6a dargestellt. Das Spektrum GS umfasst eine Vielzahl von Frequenzen, welche ein ganzzahliges Vielfaches einer Frequenz $f_0$ sind. Dabei wird das Spektrum GS durch eine kleinste Frequenz $f_{min}$ und durch eine größte Frequenz $f_{max}$ begrenzt. Sämtliche im Spektrum GS vertretenen Frequenzen weisen dieselbe Amplitude auf. Dies erleichtert die Auswertung des Ausgangssignals as, da nun Amplitudenschwankungen im Spektrum des Ausgangssignals AS unmittelbar auf das Übertragungsverhalten des Mikrowellenresonators 21 zurückzuführen sind.

Das zum Spektrum GS der Figur 6a gehörige Generatorsignal gs ist in der Figur 6b dargestellt. Ein derartiges Generatorsignal gs ist mittels moderner Signalgeneratoren ohne weiteres erzeugbar. Dabei können die Grundfrequenz $f_0$, die kleinste Frequenz $f_{min}$ und die größte Frequenz $F_{max}$ als Einstellwerte vorgegeben werden.

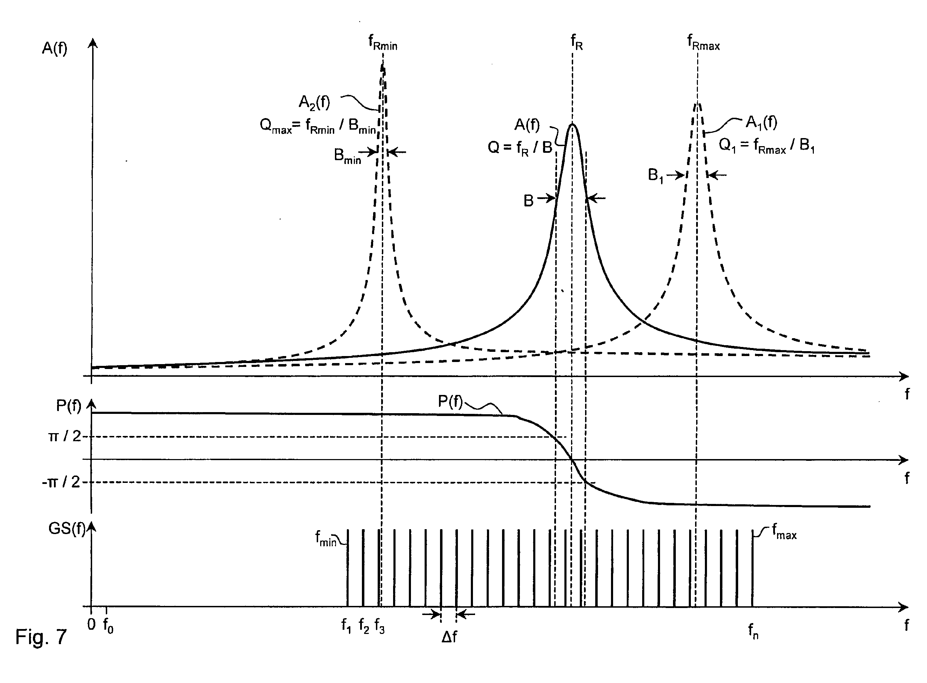

Figur 7 illustriert das Amplitudenübertragungsverhalten und das Phasenübertragungsverhalten eines Mikrowellenresonators 21 sowie ein Spektrum GS eines für die vorliegende Erfindung besonders geeigneten Generatorsignals gs. Im oberen Teil des Diagramms ist die Amplitudenübertragungsfunktion A(f) eines Mikrowellenresonators 21 für einen bestimmten Zeitpunkt dargestellt. Die Amplitudenübertragungsfunktion (A(f) ist durch die Resonanzfrequenz $f_R$ sowie durch die Bandbreite B gekennzeichnet. Die Resonanzfrequenz $f_R$ ist jene Frequenz, bei der die Amplitudenübertragungsfunktion A(f) ihr Maximum aufweist. Die Bandbreite B ist jener Frequenzbereich, in dem der Wert der Amplitudenübertragungsfunktion A(f) wenigstens den $1/\sqrt{2}$ -ten Teil des Maximums erreicht. Die Güte Q des Mikrowellenresonators 21 kann in einfacher Weise ermittelt werden, wenn die Resonanzfrequenz $f_R$ durch den Wert der Bandbreite geteilt wird. Sowohl die Resonanzfrequenz $f_R$ als auch die Bandbreite B und damit auch die Güte Q sind zeitabhängige Größen. Sie hängen sowohl von der Masse des im Messraum 22 des Mikrowellenresonators 21 befindlichen Fasergemenges FG als auch von dessen Feuchteanteil ab.

Auch aus der Phasenübertragungsfunktion P(f) kann die Resonanzfrequenz $f_R$ und die Bandbreite B bestimmt werden. So kann die Resonanzfrequenz $f_R$ mit hoher Genauigkeit bestimmt werden, indem die Nullstelle in der Phasenübertragungsfunktion P ermittelt wird. Weiterhin kann die Bandbreite B ermittelt werden, indem derjenige Frequenzbereich ermittelt wird, in dem die Phasenverschiebung höchstens +/- 45° beträgt.

Im unteren Teil des Diagramms ist das Spektrum GS eines besonders geeigneten Generatorsignals gs dargestellt. Es handelt sich um ein bandbegrenztes äquidistantes Linienspektrum. Es zeichnet sich dadurch aus, dass die Bandbreite B den Abstimmbereich des Mikrowellenresonators überdeckt. Das bedeutet, dass die kleinste Frequenz $f_{min}$ kleiner als die minimale Resonanzfrequenz $f_{Rmin}$ und die größte Frequenz $f_{max}$ größer als die größte Resonanzfrequenz $f_{Rmax}$ des Mikrowellenresonators 21 ist. Die maximale Resonanzfrequenz $f_{Rmax}$ stellt sich dann ein, wenn der Messraum 22 des Mikrowellenresonators 21 vollständig entleert ist. Das Amplitudenübertragungsverhalten des entleerten Mikrowellenresonators 21 ist als gestrichelt dargestellte Kurve $A_1$(f) dargestellt.

Die minimale Resonanzfrequenz $f_{Rmin}$ stellt sich im Betrieb einer erfindungsgemäßen Vorrichtung dann ein, wenn der Messraum 22 mit einem im wesentlichen trockenen Fasermaterial maximal gefüllt ist. In diesem Fall stellt sich auch die minimale Bandbreite $B_{min} = f_{Rmin} / Q_{max}$ ein. Der Linienabstand ΔF im Spektrum GS liegt ungefähr in der Größenordnung der minimalen Bandbreite $B_{min}$. Hierdurch ist sichergestellt, dass bei jeder möglichen Resonanzfrequenz $f_R$ und bei jeder möglichen Bandbreite B Spektrallinien im Bereich der jeweiligen Bandbreite B des Mikrowellenresonators 21 liegen. Dies ermöglicht eine genaue Bestimmung der Amplitudenübertragungsfunktion A(f) und/oder der Phaseübertragungsfunktion P(f).

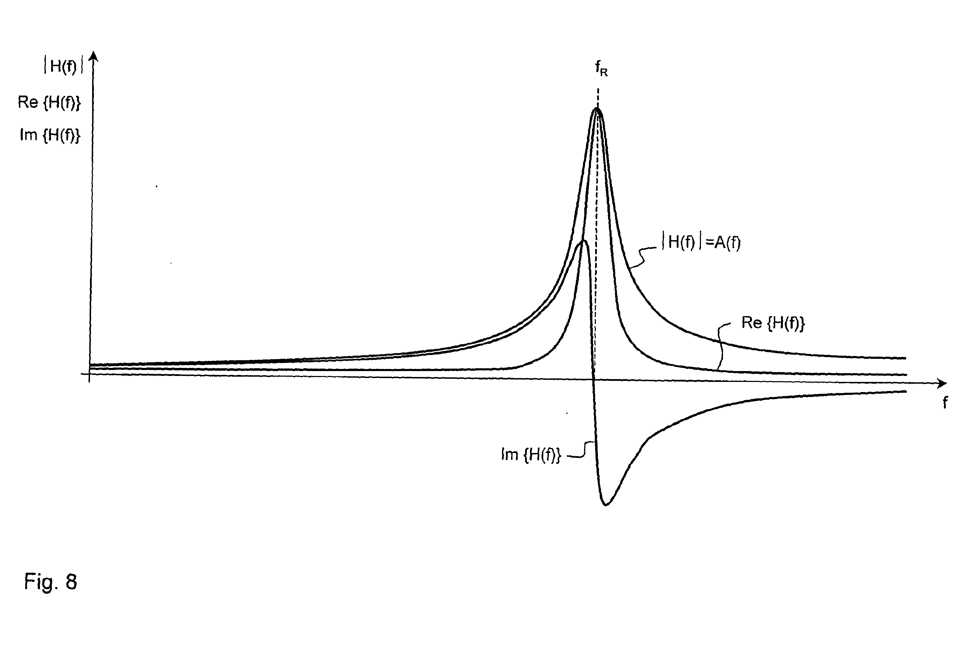

Figur 8 zeigt den Betrag, den Realteil und den Imaginärteil einer komplexen Übertragungsfunktion H(f). Die komplexe Übertragungsfunktion H(f) beschreibt sowohl das Amplitudenübertragungsverhalten als auch das Phasenübertragungsverhalten des Mikrowellenresonators 21. Der Betrag |H(f)| der komplexen Übertragungsfunktion H(f) entspricht der in der Figur 7 gezeigten Amplitudenübertragungsfunktion A(f). Sowohl der Realteil Re {H(f)} der komplexen Übertragungsfunktion H(f) als auch der Imaginärteil Im {H(f)} der komplexen Übertragungsfunktion H(f) kann aus der Betragsfunktion A(f) und der Angabe des Phasenwinkels P(f) berechnet werden. Technisch ist die Angabe der komplexen Übertragungsfunktion H(f) einerseits und der Amplitudenübertragungsfunktion A(f) und der Phasenübertragungsfunktion P(f) andererseits gleichbedeutend. Jedoch ist die komplexe Übertragungsfunktion H(f) in vielen Fällen günstiger für weitere Berechnungen im Rahmen einer Signalauswertung.

Die komplexe Übertragungsfunktion H(f) des Mikrowellenresonators 21 ist durch

$$H(f) = \frac{K}{1+jQ\,(f\,/\,f_R - f_R\,/\,f)} \qquad \text{Gleichung (1)}$$

gegeben, wobei K eine dimensionsbehaftete Konstante ist.

Der Betrag der komplexen Übertragungsfunktion H(f) ist damit durch

$$|H(f)| = \frac{K}{\sqrt{1+Q^2\,(f\,/\,f_R - f_R\,/\,f)^2}} \qquad \text{Gleichung (2)}$$

gegeben.

Weiterhin ist der Realteil durch

$$Re\,\{H(f)\} = \frac{K}{1+Q^2\,(f\,/\,f_R - f_R\,/\,f)^2} \qquad \text{Gleichung (3)}$$

und der Imaginärteil durch

$$Im\,\{H(f)\} = \frac{KQ\,(f\,/\,f_R - f_R\,/\,f)}{1+Q^2\,(f\,/\,f_R - f_R\,/\,f)^2} \qquad \text{Gleichung (4)}$$

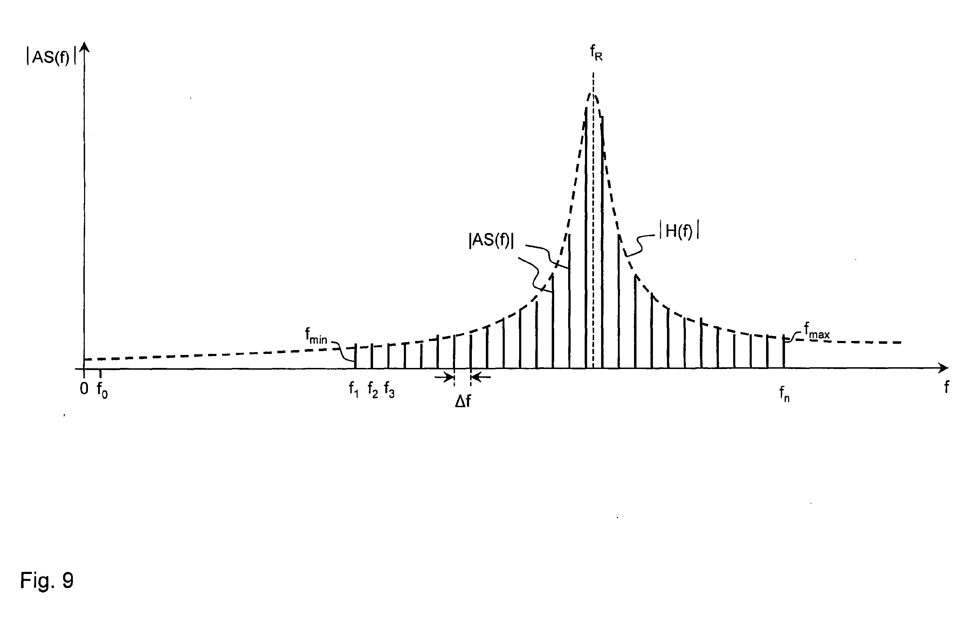

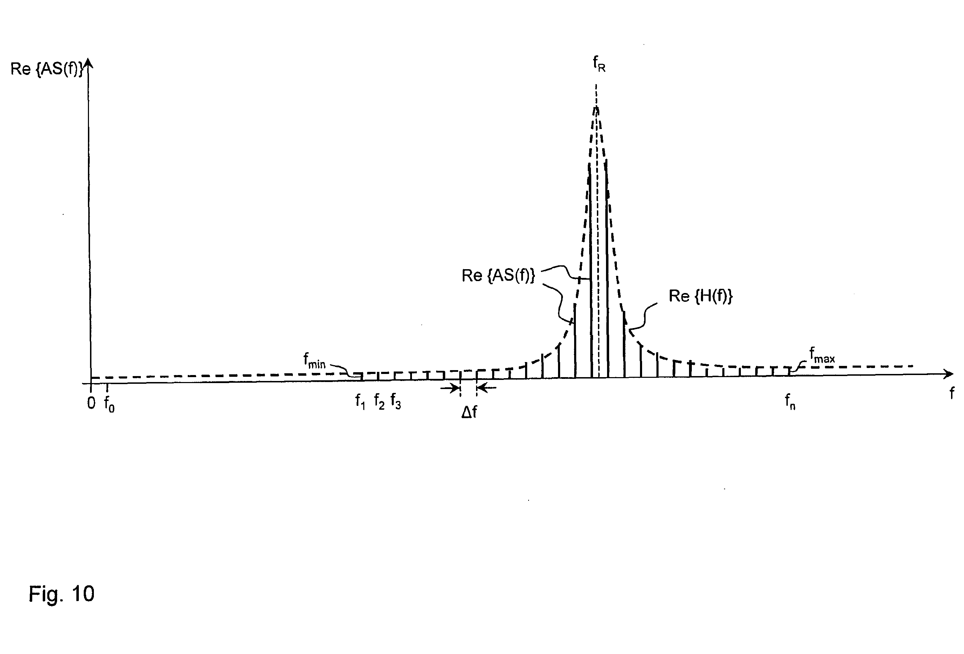

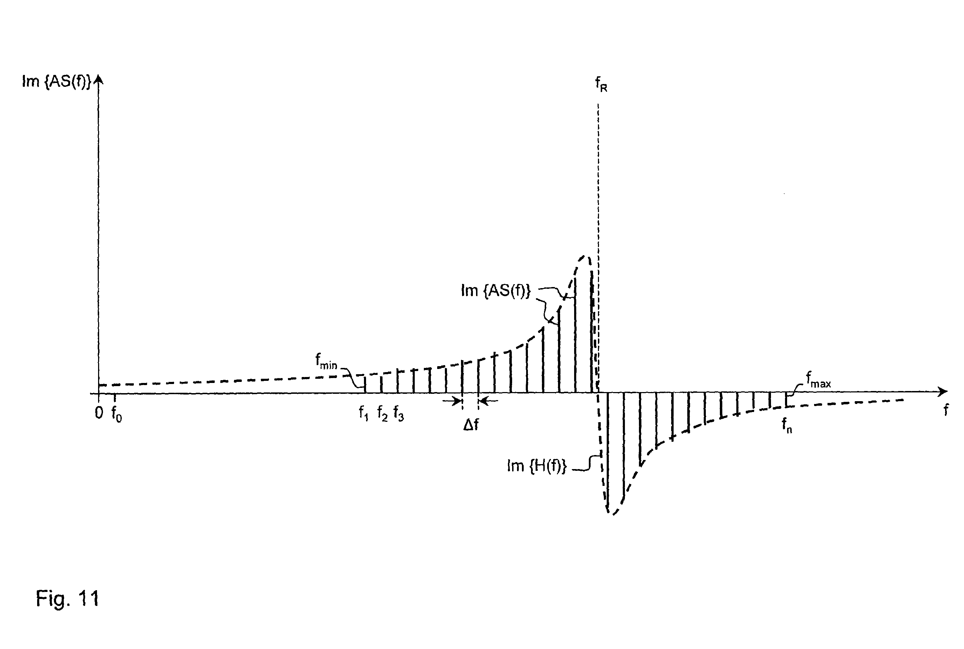

gegeben. Für ein Generatorsignal gs(t) mit einem Spektrum GS(f) nach Figur 7 und einer komplexen Übertragungsfunktion H(f) nach Figur 8 erhält man das Ausgangssignal as(t) mit dem in Figur 9 dargestellten AmplitudenBetragsspektrum |AS(f)|, mit dem in Figur 10 dargestellten Realteil Re {AS(f)} und mit dem in Figur 11 dargestellten Imaginärteil Im {AS(f)} des komplexen Amplitudenspektrums AS(f).

Da die Hüllkurve des Spektrums GS(f) im Frequenzbereich von der kleinsten Frequenz $f_{min}$ bis zu der größten Frequenz $f_{max}$ konstant ist, entspricht die Hüllkurve des Amplitudenbetragsspektrums |AS(f)| dem Betrag |H(f)|der Übertragungsfunktion H(f). Zur Veranschaulichung ist der Betrag |H(f)|der Übertragungsfunktion H(f) in Figur 9 gestrichelt dargestellt. Ebenso entspricht die Hüllkurve des Realteiles Re {AS(f)} des Spektrums AS(f) in Figur 10 und der Imaginärteil Im {A(f)} des Spektrums A(f) in Figur 11 der Hüllkurve des Realteiles Re {H(f)} der komplexen Übertragungsfunktion bzw. dem Imaginärteil Im {H(f)}der komplexen Übertragungsfunktion.

Für den Fall, dass die Hüllkurve des Spektrums GS(f) im Frequenzbereich von $f_{min}$ bis $f_{max}$ nicht konstant ist, kann die komplexe Übertragungsfunktion H(f) bestimmt werden, indem die Hüllkurve des Spektrums AS(f) durch die Hüllkurve des Spektrums GS(f) dividiert wird. Da die Hüllkurve des Spektrums GS(f) bekannt ist, kann diese Division durch Bewertung der Spektrallinien mit einem Gewichtsfaktor 1 / GS(f) erfolgen.

Die Auswertung der in den Figuren 9, 10 und 11 dargestellten Linienspektren ermöglichen die Rekonstruktion der komplexen Übertragungsfunktion H(f) des Mikrowellenresonators 21 und damit die Bestimmung von dessen Resonanzfrequenz $f_R$ und von dessen Güte Q. Die komplexe Übertragungsfunktion H(f) ist dabei in den durch die Spektrallinien des Spektrums AS(f) gegebenen Frequenzpunkten bekannt. Damit können die Beträge der einzelnen Spektrallinien unmittelbar in die Gleichungen 1, 2, 3 und 4 eingesetzt werden.

Obwohl die Resonanzfrequenz $f_R$ und die Güte Q des Mikrowellenresonators 21 auch nur aus dem Amplitudenbetragsspektrum |AS(f)| bestimmt werden können, bietet die Auswertung des Realteiles Re {AS(f)} und des Imaginärteiles Im {AS(f)} des komplexen Amplitudenspektrums AS den Vorteil der höheren Genauigkeit, da aufgrund des Vorzeichenwechsels von Im {AS(f)} bei der Resonanzfrequenz $f_R$ insbesondere die Resonanzfrequenz $F_R$ mit sehr großer Genauigkeit bestimmt werden kann. Es ist zu berücksichtigen, dass die Genauigkeit der ermittelten Resonanzfrequenz $f_R$ wesentlich größer als der Abstand ΔF der Spektrallinien sein kann, da grundsätzlich die Übertragungsfunktion H(f) aus drei diskreten Amplitudenwerten bzw. aus zwei diskreten Amplitudenwerten, wenn zusätzlich die jeweilige Phase bestimmt wird, rekonstruiert werden kann. Aufgrund unvermeidlicher Messfehler bei der Bestimmung der Abtastwerte ist es jedoch von Vorteil, wenn die Anzahl der ausgewerteten Spektrallinien erhöht wird.

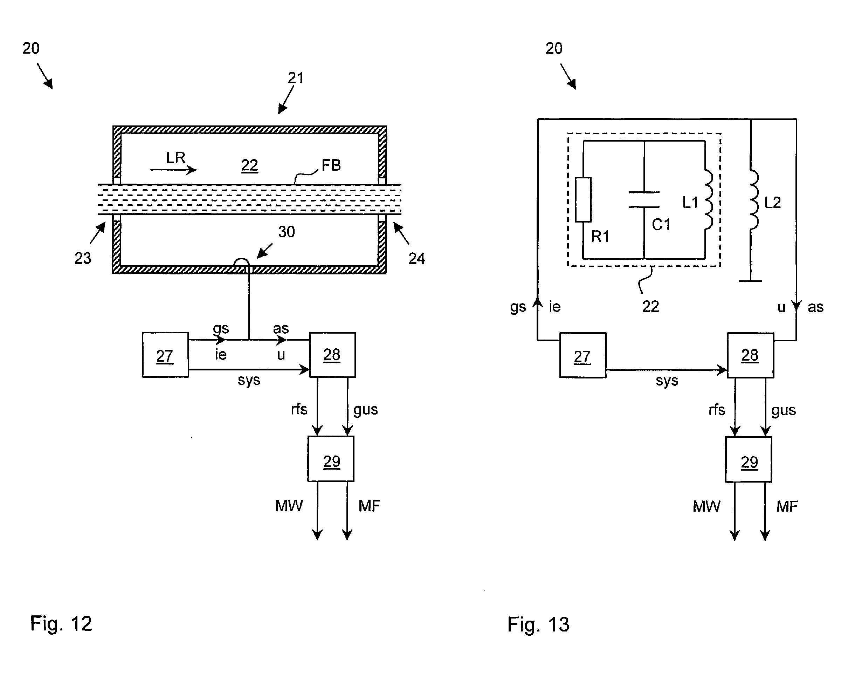

Figur 12 zeigt eine weitere erfindungsgemäße Vorrichtung 20. Der Mikrowellenresonator 21 ist hier als Eintor-Resonator mit einer Koppelanordnung 30 ausgebildet. Das von dem Generator 27 erzeugte Generatorsignal gs ist ein eingeprägter Strom ie, der über die Koppelanordnung 30 in den Mikrowellenresonator 21 eingekoppelt wird. Hierdurch entsteht an der Einkoppelanordnung 30 eine Spannung u, die als reflektiertes Ausgangssignal der Auswerteschaltung 28 zugeführt wird.

Figur 13 zeigt das Ersatzschaltbild der Vorrichtung 20 der Figur 12.

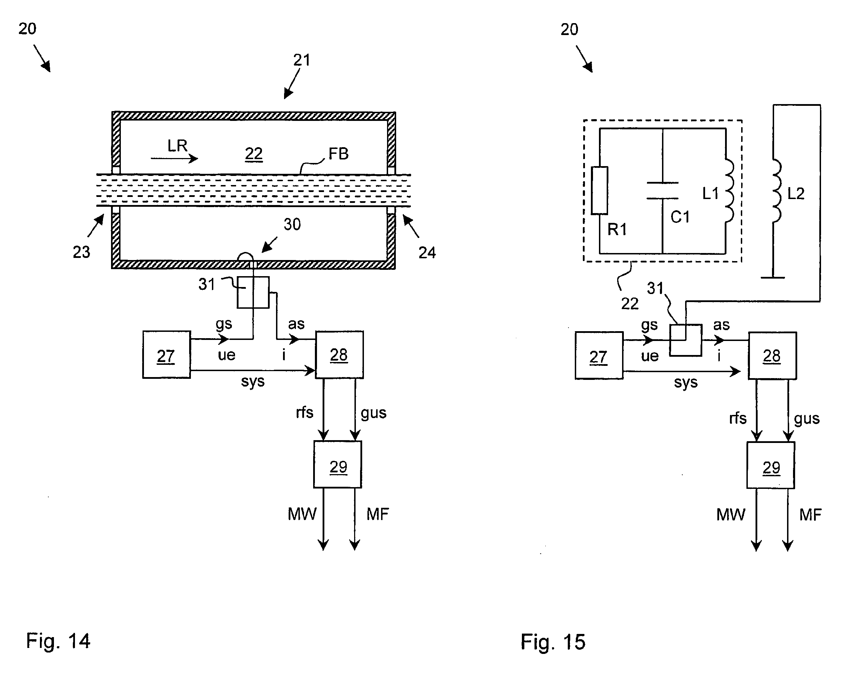

In Figur 14 ist eine weitere Ausführungsform einer erfindungsgemäßen Vorrichtung 20 dargestellt. Der Generator 27 ist so ausgebildet, dass sein Generatorsignal gs eine eingeprägte Spannung ue ist. Diese eingeprägte Spannung ue wird der Koppelanordnung 30 zugeführt. Mittels eines Stromdetektors 31 wird dann der in die Koppelanordnung 30 hineinfließende Strom i detektiert. Die gemessenen Werte des Stromes i bilden dann das Ausgangssignal des Mikrowellenresonators 21, welches dann der Auswerteschaltung 28 zur weiteren Auswertung zugeführt wird.

Figur 15 zeigt das Ersatzschaltbild der Vorrichtung 20 der Figur 14.

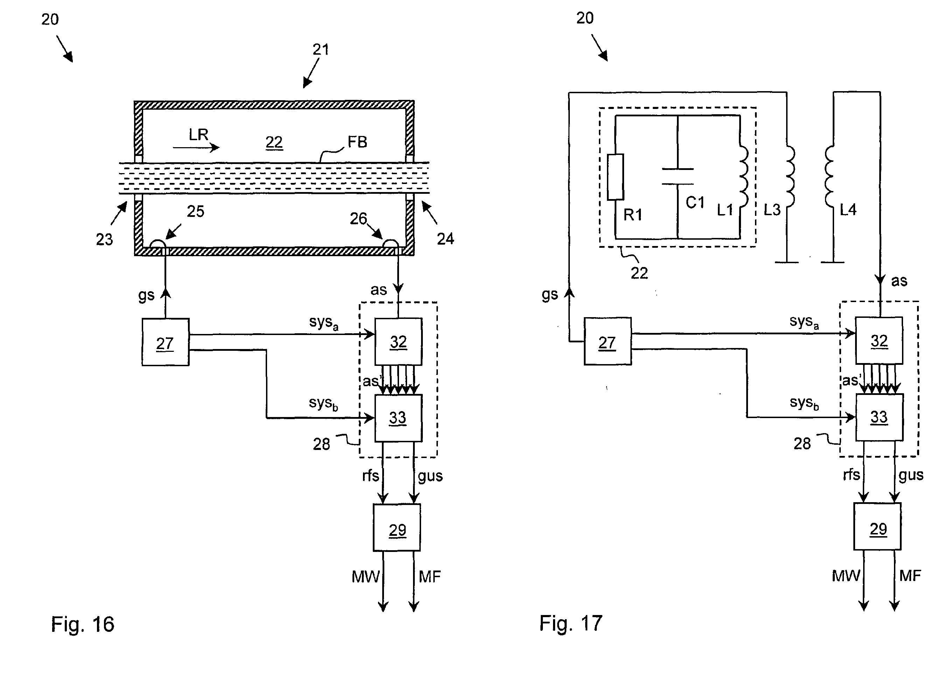

Figur 16 zeigt eine vorteilhafte Ausführungsform der Erfindung. Die Auswerteschaltung 28 besteht hier aus der Abtast- und Analog-Digital-WandlerSchaltung 32 und der digitalen Auswerteschaltung 33. Während jedes Grundintervalls der Zeitdauer To werden dem Ausgangssignal as(t) des Mikrowellenresonators 21 N Abtastwerte in den zeitlichen Abständen $N/T_0$ entnommen. Diese N Abtastwerte werden für jede Grundperiode in der digitalen Auswerteschaltung 33 zunächst Fourier-transformiert. Es ist bevorzugt, eine sogenannte schnelle Fourier-Transformation durchzuführen. Für die effiziente und schnelle Durchführung der schnellen Fourier-Transformation ist es vorteilhaft, wenn die Anzahl M der Abtastwerte pro Grundintervall durch $M = 2^k$ gegeben ist, wobei k eine positive ganze Zahl ist.

Aus dem digital vorliegenden Fourier-Spektrum, welches durch die Abtastwerte $AS(f_k)$ repräsentiert ist, wobei $f_k$ die N Frequenzen der Spektrallinien des verwendeten Generatorsignals gs(t) sind, werden durch Lösung einer oder mehrerer der Gleichungen 1 - 4 die Werte $f_R$ und Q bestimmt. Das Frequenzsignal rfs repräsentiert die (zeitlich variable) Resonanzfrequenz $f_R(t)$, und das Ausgangssignal gus repräsentiert die (zeitlich variable) Güte Q(t). Die Ausgangssignale können in digitaler und/oder analoger Form vorliegen.

Wird nur der Betrag $|H(f_k)|$der Übertragungsfunktion $H(f_k)$ durch Messung bestimmt, so erhält man aus der Gleichung 2 für jede der N Frequenzstützstellen eine Gleichung, also insgesamt N Gleichungen zur Bestimmung von $f_R$ und Q. Wird die komplexe Gleichung 1 oder werden die beiden reellen Gleichungen 3 und 4 verwendet, so erhält man insgesamt 2N reelle Gleichungen zur Bestimmung von $f_R$ und Q. Da zur Lösung des Gleichungssystems auch die (für die weitere Signalverarbeitung nicht benötigte) Konstante K bestimmt wird, werden bei Messung des Amplitudenbetrages mindestens drei Frequenzstützstellen, bei der Messung von Amplitude und Phase bzw. Realund Imaginärteil der Übertragungsfunktion mindestens zwei Stützstellen benötigt.

Zur Erzielung hoher Genauigkeit ist es vorteilhaft, die Anzahl N der Stützstellen größer als

$$Q_{max} (f_{max} - f_{min}) / f_{min}$$

zu wählen. Wenn die 3dB-Bandbreite B des Mikrowellenresonators 21 klein im Verhältnis zum Intervall $f_{max}$ - $f_{min}$ ist, so wird dabei eine entsprechend große Anzahl von Stützstellen zu verwenden sein. Das führt dazu, dass das Gleichungssystem zur Bestimmung von $f_R$ und Q überbestimmt ist und keine exakte Lösung hat. Es ist vielmehr unter der Bedingung eines (eventuell gewichteten) minimalen Fehlerbetrages oder Fehlerquadrates zu lösen. Derartige Lösungsmethoden und Algorithmen zur numerischen Lösung im Rahmen der Signalprozessierung sind bekannt.

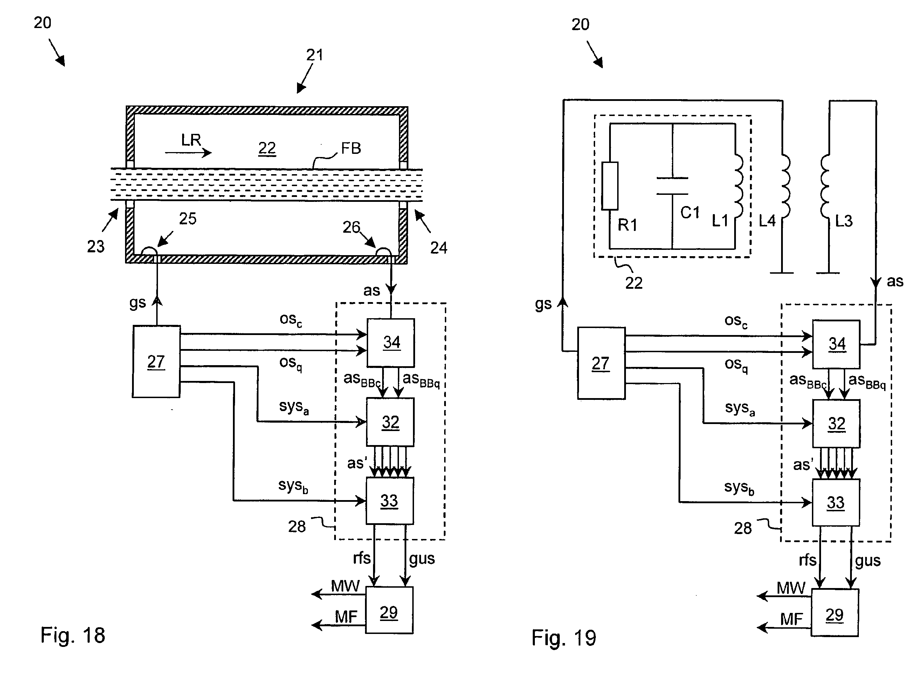

Figur 18 zeigt eine weitere beispielhafte Ausführungsform einer erfindungsgemäßen Vorrichtung 21. Die Auswerteschaltung 6 umfasst hier eine als Mischer 34 ausgebildete Transponierschaltung 34, eine Abtast- und Analog-Digital-Wandler-Schaltung 32 und eine digitale Analyseschaltung 33. Im Unterschied zur Anordnung nach Figur 16 wird in der Anordnung nach Figur 18 das Ausgangssignal as(t) des Mikrowellenresonators 21 zuerst im Mischer 34 in ein niederfrequentes Basisband transponiert. In vorteilhafter und bekannter Weise erfolgt die Frequenzumsetzung durch Mischung mit den vom Signalgenerator 27 abgegebenen Signalen

$$os_c (t) = A \cos (2\pi (n_{min} - 1) f_0 t), \quad os_q (t) = A \sin (2\pi (n_{min} - 1) f_0 t),$$

wobei $n_{min} = f_{min}/f_0$ gilt, so dass am Ausgang das Kophasalsignal $as_{BBc}$ und das Quadratursignal $as_{BBq}$ vorliegen. Dadurch, dass die zu digitalisierenden Signale im Basisband vorliegen, wird die Digitalisierung vereinfacht. Die in das Basisband transponierten Signale $as_{BBc}$ und $as_{BBq}$ haben ein Linienspektrum mit N äquidistanten Spektrallinien bei den Frequenzen von $f_0$ bis $Nf_0$. Aus dem Kophasalsignal $as_{BBc}$ und dem Quadratursignal $as_{BBq}$ werden in der digitalen Analyseschaltung 33 Real- und Imaginärteil des FourierSpektrums von H(f) bestimmt und daraus die Ausgangssignale rfs(t) und gus(t) gebildet, welche die Resonanzfrequenz $f_R(t)$ und Güte Q(t) repräsentieren.

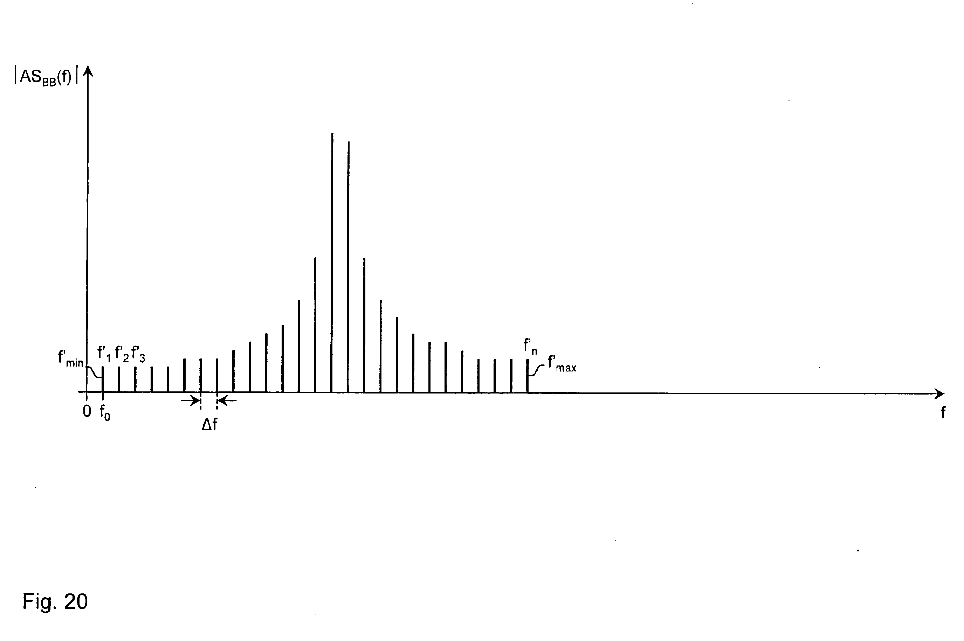

Figur 20 zeigt exemplarisch ein in ein niederfrequentes Basisband transponiertes Betragsspektrum $|AS_{BB}(f)|$ des Ausgangssignals as(t) mit dem in der Figur 9 gezeigten Betragsspektrum $|AS(f)|$. Durch Mischen mit einem geeigneten Signal sind die Frequenzen $f_1, f_2 ... f_n$ in die geringeren Frequenzen $f'_1, f'_2 ... f'_n$ umgesetzt. Die kleinste Frequenz $f'_{min}$ im transponierten Spektrum $|AS_{ss}(f)|$ entspricht dabei der Grundfrequenz $f_0$.

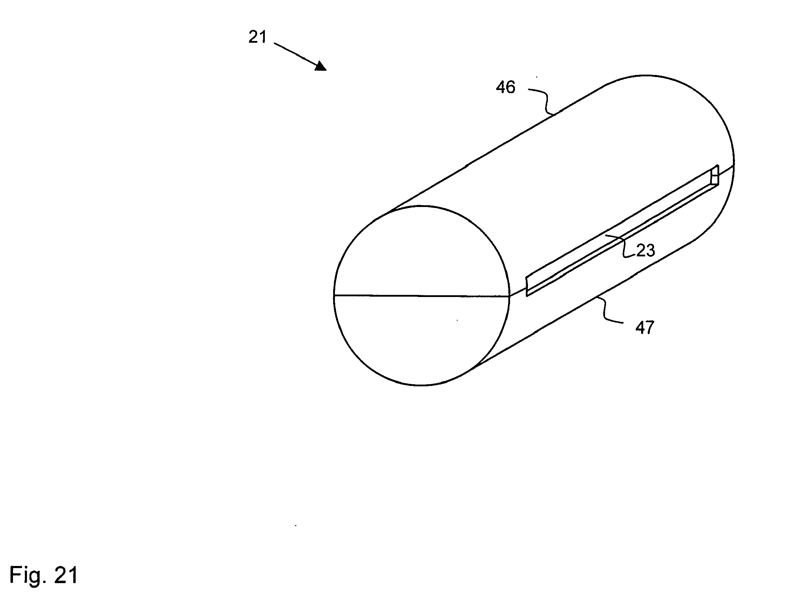

Figur 21 zeigt eine perspektivische Darstellung eines zweiteiligen Mikrowellenresonators 21. Der Mikrowellenresonator 21 umfasst ein Resonatoroberteil 46 und ein Resonatorunterteil 47, welche im Betrieb des Mikrowellenresonators 21 durch geeignete Mittel verbunden sind. Die Eingangsöffnung 23 des Mikrowellenresonators 21 ist schlitzförmig ausgebildet. Sie ist so angeordnet, dass bei dem zur Messung verwendeten Schwingungsmodus des Mikrowellenresonators 21 die elektrischen Feldlinien des elektromagnetischen Feldes parallel zur Eingangsöffnung 23 sind, wobei die magnetischen Feldlinien dazu normal verlaufen. Hierdurch ist sichergestellt, dass eine Abstrahlung des elektromagnetischen Feldes über die Eingangsöffnung 23 minimiert ist. Die in der gezeigten Perspektive nicht sichtbare Ausgangsöffnung 24 liegt der Eingangsöffnung 23 gegenüber. Auch hier sind die elektromagnetischen Feldlinien parallel zur Ausgangsöffnung 24.

Die vorliegende Erfindung ist nicht auf die dargestellten und beschriebenen Ausführungsbeispiele beschränkt. Es sind Abwandlungen im Rahmen der Patentansprüche jederzeit möglich.

Die erfindungsgemäße Vorrichtung kann insbesondere zur Ermittlung der Faserbanddichte bzw. der längenspezifischen Masse des Faserbandes unter Berücksichtigung der Feuchte Verwendung finden. Die Vorrichtung kann zur Durchführung eines Algorithmus zur Trennung von Feuchte und Dichte anhand der Güte und der Frequenz des Mikrowellenresonators ausgebildet sein.

Der Mikrowellenresonator kann zumindest bereichsweise Materialien umfassen, die für Mikrowellen durchlässig sind (Keramik, Quarzglas) und deren Dielektrizitätekonstanten möglichst temperaturunabhängig sind. Dies betrifft insbesondere im Innern des Mikrowellenresonators angeordnete Elemente zum Führen des zu vermessenden Faserbandes.

Weiterhin kann der Mikrowellenresonator aus zwei im wesentlichen parallel angeordneten Halbzylindern ausgebildet sein. Dabei kann er, insbesondere wenn er für den Auslauf einer Karde oder Stecke vorgesehen ist, als Zylinder mit konzentrischer Durchführung für das Faserband ausgebildet sein. Er kann ferner, insbesondere wenn er für den Einlauf einer Stecke vorgesehen ist, einen Einführschlitz zum seitlichen Einführen des Faserbandes aufweisen.

Um eine Verfälschung der durch die Vorrichtung erzeugten Messwerte durch Temperatureinflüsse zu vermeiden, können folgende Maßnahmen vorgesehen sein: Ausbilden des Resonators aus Materialien mit geringem Wärmeausdehungskoefffizient (z.B Invar), Vorsehen von Mitteln zur thermischen Isolation des Resonators bzw. des gesamten Sensors einschließlich zumindest eines Teiles der an den Resonator angeschlossenen Beschaltung und/oder Vorsehen von Mitteln zur Beheizung (z. B. durch Heizfolien) oder Kühlung (z. B. durch Peltierelemente) des Resonators bzw. des Sensors. Weiterhin können folgende Maßnahmen zur Kalibrierung der erfindungsgemäßen Vorrichtung vorgesehen sein: Durchführung der Kalibration des Sensors für verschiedene Faserbandmaterialien oder Faserbandmischungen anhand von Kalibrationskurven, die in Labormessungen in einer vorbetrieblichen Phase durch Ermittlung der Istbandfeinheit und/oder Ermittlung der Istfeuchte ermittelt wurden, Leeresonanzbestimmung, wobei Mittel verwendet werden, mit dem der Resonator vollständig von Faserband und eventuell angelagerten Schmutzresten gereinigt wird (Herausfördern des Faserbandes nach vorherigem Trennen, Ausblasen der Reste und Verschmutzungen).

Die erfindungsgemäße Textilmaschine kann insbesondere eine Karde oder Strecke sein, wobei eine erfindungsgemäße Vorrichtung als Auslaufsensor vorgesehen ist. Dabei kann die Vorrichtung am Streckwerksauslauf zwischen Auslaufzylinder und Kalanderwalzenpaar oder unmittelbar nach dem Kalanderwalzenpaar angeordnet sein. Mit Vorteil ist die Vorrichtung mit einem Qualitätsüberwachungssystem verbunden, wobei eine Absinken der Qualität des auslaufenden Faserbandes unter einen Schwellwert zu einer Abschaltung der Textilmaschine führt.

Insbesondere, wenn die erfindungsgemäße Textilmaschine eine Strecke ist, so kann eine erfindungsgemäße Vorrichtung als Einlaufsensor vorgesehen sein. Diese Vorrichtung kann unmittelbar vor dem Streckwerk oder in einem Einlaufbereich angeordnet und mit einer Reguliereinrichtung zum Ausregulieren von Schwankungen der längenspezifischen Masse des zu verstreckenden Faserbandes verbunden sein.

**Patentansprüche**

1. Vorrichtung (20, 20') für eine Textilmaschine (1), insbesondere für eine Spinnereivorbereitungsmaschine (1), beispielsweise für eine Strecke (1), Karde oder Kämmmaschine, oder für eine Spinnereimaschine, beispielsweise für eine Spinnmaschine oder Spulmaschine, zur Messung der längenspezifischen Masse und/oder der Feuchtigkeit eines strangförmigen, laufenden Fasergemenges (FB), mit einem Mikrowellenresonator (21), welcher einen Messraum (22) aufweist, durch den das Fasergemenge (FB) hindurchführbar ist, und mit einer Anordnung zur Erfassung der Resonanzfrequenz ($f_R$) und/oder der Güte (Q) des Mikrowellenresonators (21, 21'), <u>wobei</u> ein Signalgenerator (27) vorgesehen ist, der zur Erzeugung eines Generatorsignals (gs) ausgebildet ist, dessen Spektrum (GS) mehrere Frequenzen ($f_1$, $f_2$.... , $f_n$) umfasst, sodass das Generatorsignal (gs) in den Mikrowellenresonator (21) eingekoppelt ist und wobei ein aus dem Mikrowellenresonator (21) ausgekoppeltes Ausgangssignal (as) einer Auswerteschaltung (28) zugeführt ist, welche zur simultanen Bestimmung der Amplituden und/oder der Phase mehrerer der besagten Frequenzen ($f_1$, $f_2$, ... , $f_n$) im Spektrum (AS) des Ausgangssignals (as) ausgebildet ist, **dadurch gekennzeichnet, dass** die Auswerteschaltung (28) eine Analyseschaltung (33) zur Spektralanalyse eines durch eine Abtastschaltung (32) erzeugten Abtastsignals (as'), welches mit dem Ausgangssignal (as) korrespondiert, aufweist.

2. Vorrichtung (20, 20') nach vorstehendem Anspruch, **dadurch gekennzeichnet, dass** ein Synchronsignal (sys, $sys_a$, $sys_b$) vorgesehen ist, um die Auswerteschaltung (28) mit dem Signalgenerator (27) zu synchronisieren.

3. Vorrichtung (20, 20') nach einem der vorstehenden Ansprüche, **dadurch gekennzeichnet, dass** der Signalgenerator (27) so ausgebildet ist, dass das Spektrum (GS) des Generatorsignals (gs) durch eine vorgebbare kleinste Frequenz ($f_{min}$) und/oder durch eine vorgebbare größte Frequenz ($f_{max}$) begrenzt ist.

4. Vorrichtung (20, 20') nach Anspruch 3, **dadurch gekennzeichnet, dass** der Signalgenerator (27) so ausgebildet ist, dass die kleinste Frequenz ($f_{min}$) kleiner als die minimale betriebliche Resonanzfrequenz ($f_{Rmin}$) des Mikrowellenresonators (21) und/oder dass die größte Frequenz ($f_{max}$) größer als die maximale Resonanzfrequenz ($f_{Rmax}$) des Mikrowellenresonators (21) ist.

5. Vorrichtung (20, 20') nach einem der vorstehenden Ansprüche, **dadurch gekennzeichnet, dass** der Signalgenerator (27) so ausgebildet ist, dass die besagten Frequenzen ($f_1$, $f_2$, ... , $f_n$) im Spektrum (GS) des Generatorsignals (gs) diskret verteilt sind.

6. Vorrichtung (20, 20') nach Anspruch 5, **dadurch gekennzeichnet, dass** der Signalgenerator (27) so ausgebildet ist, dass die besagten diskreten Frequenzen ($f_1$, $f_2$, ... , $f_n$) im Spektrum (GS) des Generatorsignals (gs) äquidistant verteilt sind.

7. Vorrichtung (20, 20') nach Anspruch 5 oder 6, **dadurch gekennzeichnet, dass** der Signalgenerator (27) so ausgebildet ist, dass der Abstand ($\Delta f$) benachbarter Frequenzen ($f_1$. $f_2$, ... , $f_n$) weniger als das 1,5-fache, bevorzugt weniger als das 1-fache, besonders bevorzugt weniger als das 0,75-fache, der minimalen Bandbreite ($B_{min}$) des

Mikrowellenresonators (21, 21') beträgt.

**8.** Vorrichtung (20, 20') nach einem der vorstehenden Ansprüche, **dadurch gekennzeichnet, dass** der Signalgenerator (27) so ausgebildet ist, dass wenigstens die zur Auswertung vorgesehenen besagten Frequenzen ($f_1$, $f_2$, ... , $f_n$) im Spektrum (GS) des Generatorsignals (gs) gleiche Amplituden aufweisen.

**9.** Vorrichtung (20, 20') nach vorstehendem Anspruch, **dadurch gekennzeichnet, dass** die Auswerteschaltung (28) zur Erzeugung eines mit der Resonanzfrequenz ($f_R$) des Mikrowellenresonators (21, 21') korrespondierenden Frequenzsignals (rfs) und/oder zur Erzeugung eines mit der Güte (Q) des Mikrowellenresonators (21, 21') korrespondierenden Gütesignals (gus) ausgebildet ist.

**10.** Vorrichtung (20, 20') nach einem der vorstehenden Ansprüche, **dadurch gekennzeichnet, dass** die Auswerteschaltung (28) zur Bestimmung der Amplitude oder der Phase von wenigstens drei der besagten Frequenzen ($f_1$. $f_2$, ... , $f_n$) im Spektrum (AS) des Ausgangssignals (as) ausgebildet ist.

**11.** Vorrichtung (20, 20') nach einem der vorstehenden Ansprüche, **dadurch gekennzeichnet, dass** die Auswerteschaltung (28) zur Bestimmung der Amplitude und der Phase von wenigstens zwei der besagten Frequenzen ($f_1$, $f_2$, ... , $f_n$) im Spektrum (AS) des Ausgangssignals (as) ausgebildet ist.

**12.** Vorrichtung (20, 20') nach einem der vorstehenden Ansprüche, **dadurch gekennzeichnet, dass** die Auswerteschaltung (28) zur Bestimmung der Resonanzfrequenz ($f_R$) und/oder der Güte (Q) des Mikrowellenresonators (21, 21') unter Anwendung eines Fehlerminimierungsverfahrens ausgebildet ist.

**13.** Vorrichtung (20, 20') nach einem der vorstehenden Ansprüche, **dadurch gekennzeichnet, dass** die Auswerteschaltung (28) zur Bestimmung der Amplitude und/oder der Phase einer innerhalb der Bandbreite (B) des Mikrowellenresonators (21, 21') gelegenen besagten Frequenz ($f_1$, $f_2$, ... , $f_n$) im Spektrum (AS) des Ausgangssignals (as) ausgebildet ist.

**14.** Vorrichtung (20, 20') nach einem der vorstehenden Ansprüche, **dadurch gekennzeichnet, dass** die Auswerteschaltung (28) zur Bestimmung der Amplitude und/oder der Phase wenigstens einer oberhalb der Resonanzfrequenz ($f_R$) des Mikrowellenresonators (21, 21') gelegenen besagten Frequenz ($f_1$, $f_2$, ... , $f_n$) im Spektrum (AS) des Ausgangssignals (as) und wenigstens einer unterhalb der Resonanzfrequenz ($f_R$) des Mikrowellenresonators (21, 21') gelegenen besagten Frequenz ($f_1$, $f_2$,..., $f_n$) im Spektrum (AS) des Ausgangssignals (as) ausgebildet ist.

**15.** Vorrichtung (20, 20') nach einem der vorstehenden Ansprüche, **dadurch gekennzeichnet, dass** die Auswerteschaltung (28) zur Berücksichtigung ungleicher Amplituden der zur Auswertung vorgesehenen besagten Frequenzen ($f_1$, $f_2$, ... , $f_n$) im Spektrum (GS) des Generatorsignals (gs) ausgebildet ist.

**16.** Vorrichtung (20, 20') nach Anspruch 15, **dadurch gekennzeichnet, dass** die Abtastschaltung (32) zum Abtasten des Ausgangssignals (as) ausgebildet ist.

**17.** Vorrichtung (20, 20') nach Anspruch 15 oder 16, **dadurch gekennzeichnet, dass** die Auswerteschaltung (28) eine Transponierschaltung (34) zum Transponieren des Ausgangssignals (as) in ein niederfrequentes Basisband aufweist, um so ein transponiertes Ausgangssignal ($as_{BBc}$, $as_{BBq}$) zu erzeugen und dass die Abtastschaltung (32) zum Abtasten des transponierten Ausgangssignals ($as_{BBc}$, $as_{BBq}$) ausgebildet ist.

**18.** Vorrichtung (20, 20') nach einem der Ansprüche 15 bis 17, **dadurch gekennzeichnet, dass** die Abtastschaltung (32) ein digitales Abtastsignal (as') erzeugt und dass die Analyseschaltung (33) als digitale Analyseschaltung (33) zur Analyse des digitalen Abtastsignals (as') ausgebildet ist.

**19.** Vorrichtung (20, 20') nach einem der Ansprüche 15 bis 18, **dadurch gekennzeichnet, dass** die Analyseschaltung (33) zur Spektralanalyse des Abtastsignals (as') auf der Basis der schnellen Fourier-Transformation und/oder auf der Basis der Prony'schen Methode ausgebildet ist.

**20.** Vorrichtung (20, 20') nach einem der vorstehenden Ansprüche, **dadurch gekennzeichnet, dass** dem Mikrowellenresonator (21, 21') eine Einkoppelanordnung (25), über die das Generatorsignal (gs) in den Mikrowellenresonator (21) eingekoppelt ist, und eine Auskoppelanordnung (26), über die das Ausgangssignals (as) aus dem Mikrowellenresonator (21) ausgekoppelt ist, zugeordnet ist.

**21.** Vorrichtung (20, 20') nach einem der vorstehenden Ansprüche, **dadurch gekennzeichnet, dass** dem Mikrowellenresonator (21, 21') eine Koppelanordnung (30) zugeordnet ist, über welche das Generatorsignal (gs) in den Mikrowellenresonator (21) eingekoppelt und als Ausgangssignal (as) ein reflektiertes Signal ausgekoppelt ist.

**22.** Vorrichtung (20, 20') nach Anspruch 21, **dadurch gekennzeichnet, dass** der Signalgenerator (27) so ausgebildet ist, dass das Generatorsignal (gs) ein eingeprägter Strom (ie) ist und dass die Auswerteschaltung (28) zur Auswertung der als reflektiertes Ausgangssignal (as) an der Koppelanordnung (30) auftretenden Spannung (u) ausgebildet ist.

**23.** Vorrichtung (20, 20') nach Anspruch 21 oder 22, **dadurch gekennzeichnet, dass** der Signalgenerator (27) so ausgebildet ist, dass das Generatorsignal (gs) eine eingeprägte Spannung (ue) ist und dass die Auswerteschaltung (28) zur Auswertung des als reflektiertes Ausgangssignal (as) in der Koppelanordnung (30) fließenden Stromes (i) ausgebildet ist.

**24.** Vorrichtung (20, 20') nach einem der Ansprüche 21 bis 23, **dadurch gekennzeichnet, dass** der Signalgenerator (27) so ausgebildet ist, dass das Generatorsignal (gs) eine eingeprägte Welle ist und dass die Auswerteschaltung (28) zur Auswertung der als reflektiertes Ausgangssignal (as) an der Koppelanordnung (30) auftretenden Amplitude und/oder Phase der Welle ausgebildet ist.

**25.** Vorrichtung (20, 20') nach einem der vorstehenden Ansprüche, **dadurch gekennzeichnet, dass** eine Signalauswertestufe (29) vorgesehen ist, welche aus dem Frequenzsignal (rfs) und/oder aus dem Gütesignal (gus) Messwerte (MW, MW') der längenspezifischen Masse und/oder Messwerte (MF) der Feuchtigkeit des strangförmigen, laufenden Fasergemenges (FB) erzeugt.

**26.** Vorrichtung (20, 20') nach einem der Ansprüche 1 bis 25, **dadurch gekennzeichnet, dass** wenigstens ein FPGA vorgesehen ist.

**27.** Vorrichtung (20, 20') nach einem der Ansprüche 1 bis 26, **dadurch gekennzeichnet, dass** der Mikrowellenresonator (21, 21') und zumindest Teile der daran angeschlossenen Schaltung (27, 28, 29), insbesondere der Signalgenerator (27), die Auswerteschaltung (28) und/oder die Signalauswertestufe (29), in einem gemeinsamen Gehäuse angeordnet sind.

**28.** Vorrichtung (20, 20') nach einem der Ansprüche 1 bis 27, **dadurch gekennzeichnet, dass** eine Kommunikationsschnittstelle zum Anbinden der Vorrichtung an eine Kommunikationsverbindung, beispielsweise an einen CAN-Bus, einer Textilmaschine (1) vorgesehen ist.

**29.** Textilmaschine (1), insbesondere eine Spinnereivorbereitungsmaschine (1), beispielsweise eine Strecke (1), Karde oder Kämmmaschine, oder eine Spinnereimaschine, beispielsweise eine Spinnmaschine oder Spulmaschine, **dadurch gekennzeichnet, dass** wenigstens eine Vorrichtung (20, 20') nach einem der vorstehenden Ansprüche vorgesehen ist.

**Claims**

**1.** Device (20, 20') for a textile machine (1), in particular for a spinning preparation machine (1), such as a draw frame (1), carder or combing machine, or for a machine in a spinning mill, such as for a spinning machine or a winder, for measuring the mass per unit length and/or moisture content of a skein-like, moving fibre batch (FB), with a microwave resonator (21) that incorporates a measuring chamber (22) through which the fibre batch (FB) can be passed, and with an arrangement for determining the resonant frequency ($f_R$) and/or the quality factor (Q-factor) of the microwave resonator (21, 21'), wherein a signal generator (27) is provided that is designed to output a generator signal (gs) whose spectrum (GS) contains a number of frequencies ($f_1$, $f_2$, ... , $f_n$), so that the generator signal (gs) is coupled into the microwave resonator (21), and an output signal (as) coupled out of the microwave resonator (21) is passed to an evaluation circuit (28) that is designed for the simultaneous determination of the amplitude and/or phase of a number of the said frequencies ($f_1$, $f_2$, ... , $f_n$) in the spectrum (AS) of the output signal (as), **characterized in that** the evaluation circuit (28) incorporates an analysis circuit (33) for spectral analysis of a sampled signal (as') generated by a sampling circuit (32), and which corresponds to the output signal (as).

**2.** Device (20, 20') according to the foregoing claim, **characterized in that** a synchronizing signal (sys, $sys_a$, $sys_b$) is provided in order to synchronize the evaluation circuit (28) with the signal generator (27).

**3.** Device (20, 20') according to one of the foregoing claims, <u>**characterized in that**</u> the signal generator (27) is designed in such a way that the spectrum (GS) of the generator signal (gs) is limited by a specifiable minimum frequency ($f_{min}$) and/or by a specified maximum frequency ($f_{max}$).

**4.** Device (20, 20') according to Claim 3, <u>**characterized in that**</u> the signal generator (27) is designed in such a way that the minimum frequency ($f_{min}$) is smaller than the minimum usable resonant frequency ($f_{Rmin}$) of the microwave resonator (21), and/or that the maximum frequency ($f_{max}$) is larger than the maximum resonant frequency ($f_{Rmax}$) of the microwave resonator (21).

**5.** Device (20, 20') according to one of the foregoing claims, <u>**characterized in that**</u> the signal generator (27) is designed in such a way that the said frequencies ($f_1, f_2, ... , f_n$) are distributed discretely across the spectrum (GS) of the generator signal (gs).

**6.** Device (20, 20') according to Claim 5, <u>**characterized in that**</u> the signal generator (27) is designed in such a way that the said discrete frequencies ($f_1, f_2, ... , f_n$) are distributed equidistantly across the spectrum (GS) of the generator signal (gs).

**7.** Device (20, 20') according to Claim 5 or 6, <u>**characterized in that**</u> the signal generator (27) is configured in such a way that the spacing ($\Delta f$) between neighbouring frequencies ($f_1, f_2, ... , f_n$) is less than 1.5 times the minimum bandwidth ($B_{min}$) of the microwave resonator (21, 21'), more favourably less than that bandwidth, and particularly favourably less than 0.75 times that bandwidth.

**8.** Device (20, 20') according to one of the foregoing claims, <u>**characterized in that**</u> the signal generator (27) is designed in such a way that at least those of the said frequencies ($f_1, f_2, ... , f_n$) in the spectrum (GS) of the generator signal (gs) that are to be used for evaluation have the same amplitudes.

**9.** Device (20, 20') according to the foregoing claim, <u>**characterized in that**</u> the evaluation circuit (28) is configured to generate a frequency signal (rfs) that corresponds to the resonant frequency ($f_R$) of the microwave resonator (21, 21') and/or to generate a quality factor signal (gus) that corresponds to the quality factor of the microwave resonator (21, 21').

**10.** Device (20, 20') according to one of the foregoing claims, <u>**characterized in that**</u> the evaluation circuit (28) is designed to determine the amplitude or phase of at least three of the said frequencies ($f_1, f_2, ... , f_n$) in the spectrum (AS) of the output signal (as).

**11.** Device (20, 20') according to one of the foregoing claims, <u>**characterized in that**</u> the evaluation circuit (28) is designed to determine the amplitude and the phase of at least two of the said frequencies ($f_1, f_2, ... , f_n$) in the spectrum (AS) of the output signal (as).

**12.** Device (20, 20') according to one of the foregoing claims, <u>**characterized in that**</u> the evaluation circuit (28) is designed to determine the resonant frequency ($f_R$) and/or the quality factor (Q) of the microwave resonator (21, 21') using an error minimization algorithm.

**13.** Device (20, 20') according to one of the foregoing claims, <u>**characterized in that**</u> the evaluation circuit (28) is designed to determine the amplitude and/or the phase of a said frequency ($f_1, f_2, ..., f_n$) in the spectrum (AS) of the output signal (as) that is within the bandwidth (B) of the microwave resonator (21, 21').

**14.** Device (20, 20') according to one of the foregoing claims, <u>**characterized in that**</u> the evaluation circuit (28) is designed to determine the amplitude and/or the phase of at least one of the said frequencies ($f_1, f_2, ... , f_n$) in the spectrum (AS) of the output signal (as) that is above the resonant frequency ($f_R$) of the microwave resonator (21, 21') and at least one of the said frequencies ($f_1, f_2, ... , f_n$) in the spectrum (AS) of the output signal (as) that is below the resonant frequency ($f_R$) of the microwave resonator (21, 21').

**15.** Device (20, 20') according to one of the foregoing claims, <u>**characterized in that**</u> the evaluation circuit (28) is designed to take into account unequal amplitudes of the said frequencies ($f_1, f_2, ... , f_n$) in the spectrum (GS) of the generator signal (gs) that are provided for evaluation.

**16.** Device (20, 20') according to claim 15, <u>**characterized in that**</u> the sampling circuit (32) is configured to sample the output signal (as).

17. Device (20, 20') according to Claim 15 or 16, **characterized in that** the evaluation circuit (28) incorporates a transposing circuit (34) to transpose the output signal (as) to a low-frequency baseband in order to generate a transposed output signal_($as_{BBc}$, $as_{BBq}$), and where the sampling circuit (32) is designed to sample the transposed output signal ($as_{BBc}$, $as_{BBq}$).

18. Device (20, 20') according to one of Claims 15 to 17, **characterized in that** the sampling circuit (32) generates a digital sampled signal (as'), and that the analysis circuit (33) is designed as a digital analysis circuit (33) for analysing the digital sampled signal (as').

19. Device (20, 20') according to one of Claims 15 to 18, **characterized in that** the analysis circuit (33) is designed for spectral analysis of the sampled signal (as'), on the basis of the fast Fourier transform and/or on the basis of Prony's method.

20. Device (20, 20') according to one of the foregoing claims, **characterized in that** an inward coupling arrangement (25) is assigned to the microwave resonator (21, 21') by means of which inward coupling arrangement (25) the generator signal (gs) is coupled into the microwave resonator (21), and an outward coupling arrangement (26) is assigned to the microwave resonator (21, 21') by means of which outward coupling arrangement (26) the output signal (as) is coupled out of the microwave resonator (21).

21. Device (20, 20') according to one of the foregoing claims, **characterized in that** a coupling arrangement (30) is assigned to the microwave resonator (21, 21') by means of which coupling arrangement (30) the generator signal (gs) is coupled into the microwave resonator (21), and through which a reflected signal is coupled out as an output signal (as).

22. Device (20, 20') according to Claim 21, **characterized in that** the signal generator (27) is designed in such a way that the generator signal (gs) takes the form of an injected current (ie), and that the evaluation circuit (28) is designed to evaluate a voltage (u) appearing at the coupling arrangement (30) as the reflected output signal (as).

23. Device (20, 20') according to Claim 21 or 22, **characterized in that** the signal generator (27) is designed in such a way that the generator signal (gs) takes the form of an impressed voltage (ue), and that the evaluation circuit (28) is designed to evaluate a current (i) flowing in the coupling arrangement (30) as the reflected output signal (as).

24. Device (20, 20') according to one of Claims 21 to 23, **characterized in that** the signal generator (27) is designed in such a way that the generator signal (gs) takes the form of an impressed wave, and that the evaluation circuit (28) is designed to evaluate the amplitude and/or phase of the wave appearing at the coupling arrangement (30) as the reflected output signal (as).

25. Device (20, 20') according to one of the foregoing claims, **characterized in that** a signal evaluation stage (29) is provided which obtains measurements (MW, MW') of the mass per unit length and/or measurements (MF) of the moisture content in the skein-like, moving fibre batch (FB) from the frequency signal (rfs) and/or from the quality factor signal (gus).

26. Device (20, 20') according to one of Claims 1 to 25, **characterized in that** at least one FPGA is provided.

27. Device (20, 20') according to one of Claims 1 to 26, **characterized in that** the microwave resonator (21, 21'), and at least part of the circuitry (27, 28, 29) connected to it, in particular the signal generator (27), the evaluation circuit (28) and/or the signal evaluation stage (29) are contained in a common housing.

28. Device (20, 20') according to one of Claims 1 to 27, **characterized in that** a communication interface is provided for connecting the device to an external communication path, such as a CAN bus, on a textile machine (1).

29. Textile machine (1), in particular a spinning preparation machine (1), for instance a draw frame (1), carder or combing machine, or a machine in a spinning mill such as a spinning machine or a winder, **characterized in that** at least one device (20, 20') according to one of the foregoing claims is provided.

**Revendications**

1. Dispositif (20, 20') pour une machine textile (1), particulièrement pour une machine de préparation de filage (1), par exemple une étireuse (1), une cardeuse ou une peigneuse, ou pour une machine de filage, par exemple un métier à filer ou une bobineuse, pour mesurer la masse spécifique à la longueur et/ou l'humidité d'un mélange de fibres (FB) en boyau continu, avec un résonateur à hyperfréquences (21) comportant un espace de mesure (22), à travers lequel le mélange de fibres (FB) peut être transporté, et avec un arrangement pour saisir la fréquence de résonance ($f_R$) et/ou la qualité (Q) du résonateur à hyperfréquences (21, 21'), sachant qu'il est prévu un générateur de signaux (27) se présentant sous une forme telle qu'il génère un signal de générateur (gs) dont le spectre (GS) comprend plusieurs fréquences ($f_1$, $f_2$,..., $f_n$), si bien que le signal de générateur (gs) est injecté dans le résonateur à hyperfréquences (21), et sachant qu'un signal de sortie (as) extrait du résonateur à hyperfréquences (21) est délivré à un circuit d'évaluation (28), qui se présente sous une forme telle qu'il détermine simultanément l'amplitude et/ou la phase de plusieurs desdites fréquences ($f_1$, $f_2$, ..., $f_n$) dans le spectre (AS) du signal de sortie (as), **caractérisé en ce que** le circuit d'évaluation (28) comporte un circuit d'analyse (33) pour l'analyse spectrale d'un signal de balayage (as') généré par un circuit de balayage (32) et correspondant au signal de sortie (as).

2. Dispositif (20, 20') selon la revendication précédente, **caractérisé en ce qu'**il est prévu un signal de synchronisation ($sys$, $sys_a$, $sys_b$) pour synchroniser le circuit d'évaluation (28) avec le générateur de signaux (27).

3. Dispositif (20, 20') selon l'une des revendications précédentes, **caractérisé en ce que** le générateur de signaux (27) se présente sous une forme telle que le spectre (GS) du signal de générateur (gs) est limité par une fréquence minimale allouable ($f_{min}$) et/ou par une fréquence maximale allouable ($f_{max}$).

4. Dispositif (20, 20') selon la revendication 3, **caractérisé en ce que** le générateur de signaux (27) se présente sous une forme telle que la fréquence minimale ($f_{min}$) est inférieure à la fréquence de résonance minimale en fonctionnement ($f_{Rmin}$) du résonateur à hyperfréquences (21) et/ou que la fréquence maximale ($f_{max}$) est supérieure à la fréquence de résonance maximale ($f_{Rmax}$) du résonateur à hyperfréquences (21).

5. Dispositif (20, 20') selon l'une des revendications précédentes, **caractérisé en ce que** le générateur de signaux (27) se présente sous une forme telle que lesdites fréquences ($f_1$, $f_2$, ..., $f_n$) soient réparties discrètement dans le spectre (GS) du signal de générateur (gs).

6. Dispositif (20, 20') selon la revendication 5, **caractérisé en ce que** le générateur de signaux (27) se présente sous une forme telle que lesdites fréquences discrètes ($f_1$, $f_2$, ..., $f_n$) soient réparties à équidistance dans le spectre (GS) du signal de générateur (gs).

7. Dispositif (20, 20') selon la revendication 5 ou 6, **caractérisé en ce que** le générateur de signaux (27) se présente sous une forme telle que l'écart ($\Delta f$) entre des fréquences voisines ($f_1$, $f_2$, ..., $f_n$) soit inférieur à 1,5 fois, de préférence inférieur à 1 fois, de manière particulièrement préférée, inférieur à 0,75 fois la largeur de bande minimale ($B_{min}$) du résonateur à hyperfréquences (21, 21').

8. Dispositif (20, 20') selon l'une des revendications précédentes, **caractérisé en ce que** le générateur de signaux (27) se présente sous une forme telle qu'au moins lesdites fréquences ($f_1$, $f_2$, ..., $f_n$) prévues pour l'évaluation présentent des amplitudes égales dans le spectre (GS) du signal de générateur (gs).

9. Dispositif (20, 20') selon la revendication précédente, **caractérisé en ce que** le circuit d'évaluation (28) se présente sous une forme permettant de générer un signal de fréquence (rfs) correspondant à la fréquence de résonance ($f_R$) du résonateur à hyperfréquences (21, 21') et/ou de générer un signal de qualité (gus) correspondant à la qualité (Q) du résonateur à hyperfréquences (21, 21').

10. Dispositif (20, 20') selon l'une des revendications précédentes, **caractérisé en ce que** le circuit d'évaluation (28) se présente sous une forme permettant de déterminer l'amplitude ou la phase de trois au moins desdites fréquences ($f_1$, $f_2$, ..., $f_n$) dans le spectre (AS) du signal de sortie (as).

11. Dispositif (20, 20') selon l'une des revendications précédentes, **caractérisé en ce que** le circuit d'évaluation (28) se présente sous une forme permettant de déterminer l'amplitude et la phase de deux au moins desdites fréquences ($f_1$, $f_2$, ..., $f_n$) dans le spectre (AS) du signal de sortie (as).

**12.** Dispositif (20, 20') selon l'une des revendications précédentes, **caractérisé en ce que** le circuit d'évaluation (28) se présente sous une forme permettant de déterminer la fréquence de résonance ($f_R$) et/ou la qualité (Q) du résonateur à hyperfréquences (21, 21') à l'aide de méthode de minimisation d'erreurs.

**13.** Dispositif (20, 20') selon l'une des revendications précédentes, **caractérisé en ce que** le circuit d'évaluation (28) se présente sous une forme permettant de déterminer l'amplitude et/ou la phase d'une fréquence ($f_1$, $f_2$, ..., $f_n$) située au sein de la largeur de bande (B) du résonateur à hyperfréquences (21, 21') dans le spectre (AS) du signal de sortie (as).

**14.** Dispositif (20, 20') selon l'une des revendications précédentes, **caractérisé en ce que** le circuit d'évaluation (28) se présente sous une forme permettant de déterminer l'amplitude et/ou la phase d'au moins une desdites fréquences ($f_1$, $f_2$, ..., $f_n$) située au-dessus de la fréquence de résonance ($f_R$) du résonateur à hyperfréquences (21, 21') dans le spectre (AS) du signal de sortie (as) et d'au moins une desdites fréquences ($f_1$, $f_2$, ..., $f_n$) située en-deçà de la fréquence de résonance ($f_R$) du résonateur à hyperfréquences (21, 21') dans le spectre (AS) du signal de sortie (as).

**15.** Dispositif (20, 20') selon l'une des revendications précédentes, **caractérisé en ce que** le circuit d'évaluation (28) se présente sous une forme permettant de tenir compte d'amplitudes inégales desdites fréquences ($f_1$, $f_2$, ..., $f_n$) prévues pour l'évaluation dans le spectre (GS) du signal de générateur (gs).

**16.** Dispositif (20, 20') selon la revendication 15, **caractérisé en ce que** le circuit d'évaluation (32) se présente sous une forme permettant d'échantillonner le signal de sortie (as).

**17.** Dispositif (20, 20') selon la revendication 15 ou 16, **caractérisé en ce que** le circuit d'évaluation (28) comporte un circuit de transposition (34) pour transposer le signal de sortie (as) dans une bande de base à basse fréquence, pour générer ainsi un signal de sortie transposé ($as_{BBc}$, $as_{BBq}$), et **en ce que** le circuit d'évaluation (32) se présente sous une forme permettant d'échantillonner le signal de sortie transposé ($as_{BBc}$, $as_{BBq}$).

**18.** Dispositif (20, 20') selon l'une des revendications 15 à 17, **caractérisé en ce que** le circuit d'évaluation (32) génère un signal d'échantillonnage numérique (as') et **en ce que** le circuit d'analyse (33) se présente sous la forme d'un circuit d'analyse numérique (33) pour l'analyse du signal d'échantillonnage numérique (as').

**19.** Dispositif (20, 20') selon l'une des revendications 15 à 18, **caractérisé en ce que** le circuit d'analyse (33) se présente sous une forme permettant l'analyse spectrale du signal d'échantillonnage (as') sur la base de la transformation de Fourier rapide et/ou de la méthode de Prony.

**20.** Dispositif (20, 20') selon l'une des revendications précédentes, **caractérisé en ce qu'**un arrangement d'injection (25), par lequel le signal de générateur (gs) est injecté dans le résonateur à hyperfréquences (21, 21), et un arrangement d'extraction (26), par lequel le signal de sortie (as) est extrait du résonateur à hyperfréquences (21), sont attribués au résonateur à hyperfréquences (21).

**21.** Dispositif (20, 20') selon l'une des revendications précédentes, **caractérisé en ce qu'**un arrangement de couplage (30), par lequel le signal de générateur (gs) est injecté dans le résonateur à hyperfréquences (21) et, comme signal de sortie (as), un signal réfléchi est extrait, est attribué au résonateur à hyperfréquences (21, 21').

**22.** Dispositif (20, 20') selon la revendication 21, **caractérisé en ce que** le générateur de signaux (27) se présente sous une forme telle que le signal de générateur (gs) soit un courant appliqué (ie), et **en ce que** le circuit d'évaluation (28) se présente sous une forme permettant l'évaluation de la tension (u) apparaissant à l'arrangement de couplage (30) comme signal de sortie réfléchi (as).

**23.** Dispositif (20, 20') selon la revendication 21 ou 22, **caractérisé en ce que** le générateur de signaux (27) se présente sous une forme telle que le signal de générateur (gs) soit une tension appliquée (ue), et **en ce que** le circuit d'évaluation (28) se présente sous une forme permettant l'évaluation du courant (i) circulant dans l'arrangement de couplage (30) comme signal de sortie réfléchi (as).

**24.** Dispositif (20, 20') selon l'une des revendications 21 à 23, **caractérisé en ce que** le générateur de signaux (27) se présente sous une forme telle que le signal de générateur (gs) soit une onde appliquée, et **en ce que** le circuit d'évaluation (28) se présente sous une forme permettant l'évaluation de l'amplitude et/ou la phase de l'onde apparaissant à l'arrangement de couplage (30) comme signal de sortie réfléchi (as).

**25.** Dispositif (20, 20') selon des revendications précédentes, **caractérisé en ce qu'**il est prévu un étage d'évaluation de signaux (29), qui, à partir du signal de fréquence (rfs) et/ou du signal de qualité (gus), génère des valeurs de mesure (MW, MW') de la masse spécifique à la longueur et/ou des valeurs de mesure (MF) d'humidité du mélange de fibres en boyau continu (FB).

**26.** Dispositif (20, 20') selon l'une des revendications 1 à 25, **caractérisé en ce qu'**il est prévu au moins un FPGA.

**27.** Dispositif (20, 20') selon l'un des revendications 1 à 26, **caractérisé en ce que** le résonateur à hyperfréquences (21, 21') et au moins des parties du circuit (27, 28, 29) qui y est raccordé, en particulier le générateur de signaux (27), le circuit d'évaluation (28) et/ou l'étage d'évaluation des signaux (29), sont logés dans un boîtier commun.

**28.** Dispositif (20, 20') selon l'une des revendications 1 à 27, **caractérisé en ce qu'**il est prévu une interface de communication pour rattacher le dispositif à une liaison de communication, par exemple un bus CAN, d'une machine textile (1).

**29.** Machine textile (1), en particulier une machine de préparation de filage (1), par exemple une étireuse (1), une cardeuse ou une peigneuse, ou une machine de filage, par exemple un métier à filer ou une bobineuse, **caractérisée en ce qu'**il est prévu au moins un dispositif (20, 20') selon l'une des revendications précédentes.

Fig. 1

SdT

Fig. 2

Fig. 3

Fig. 4

GS(f)

-3  -2  -1  0  1  2  3   f / f₀

Fig. 5a

gs(t)

-3  -2  -1  0  1  2  3   t / T₀

Fig. 5b

GS(f)

f₀    f_min        f_max   f

Fig. 6a

gs(t)

t

Fig. 6b

Fig. 7

Fig. 8

EP 2 069 777 B1

Fig. 9

EP 2 069 777 B1

Fig. 10

EP 2 069 777 B1

Fig. 11

Fig. 12

Fig. 13

Fig. 15

Fig. 14

Fig. 17

Fig. 16

Fig. 19

Fig. 18

Fig. 20

Fig. 21

**EP 2 069 777 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6476619 B

- DE 19705260